# EUROPEAN PATENT SPECIFICATION

(11) **EP 4 114 204 B1**
(45) Date of publication and mention of the grant of the patent: **02.09.2026**
(21) Application number: 21763749.5
(22) Date of filing: 03.03.2021
(51) Int. Cl.: A23L 33/105, A61K 36/185, A61K 36/324, A61P 25/00, A61P 25/28, A23L 33/115

(54) **SYNERGISTIC COMPOSITIONS FOR IMPROVING BRAIN HEALTH**
SYNERGISTISCHE ZUSAMMENSETZUNGEN ZUR VERBESSERUNG DER GEHIRNGESUNDHEIT
COMPOSITIONS SYNERGIQUES POUR AMÉLIORER LA SANTÉ CÉRÉBRALE

(30) Priority: 03.03.2020 IN 202041008980
(43) Date of publication of application: 11.01.2023
(73) Proprietor: Laila Nutra Private Limited, Kanuru, Vijaywada - 520007 (IN)
(72) Inventor: GOKARAJU, Ganga Raju, Vijayawada, Andhra Pradesh 520010 (IN); BHUPATHIRAJU, Kiran, Vijayawada, Andhra Pradesh 520010 (IN); GOKARAJU, Rama Raju, Vijayawada, Andhra Pradesh 520010 (IN); GOLAKOTI, Trimurtulu, Vijayawada, Andhra Pradesh 520 010 (IN); GOKARAJU, Venkata Kanaka Ranga Raju, Vijayawada, Andhra Pradesh 520010 (IN); SOMEPALLI, Venkateswarlu, Vijayawada, Andhra Pradesh 520 010 (IN); ALLURI, Venkata Krishna Raju, Vijayawada, Andhra Pradesh 520 010 (IN); SENGUPTHA, Krishanu, Vijayawada, Andhra Pradesh 520 010 (IN)
(74) Representative: Isarpatent
(86) International application number: PCT/IN2021/050201
(87) International publication number: WO 2021/176469

(56) References cited:
- WO-A1-2015/118557
- WO-A1-2020/089944
- WO-A2-2011/114350
- WO-A2-2012/094636
- US-A1- 2010 112 101
- US-A1- 2012 301 432
- US-A1- 2016 339 072
- US-A1- 2017 136 076
- US-A1- 2017 136 076
- "Majoon Bara-e- Nisyaan", TKDL,, 1 January 1974 (1974-01-01), XP003029404
- ANONYMOUS: "Arq-e- Gazar Anbari Ba Nuskha Kalan", TKDL,, 1 January 1938 (1938-01-01), XP003031094
- VREESE ET AL.: "Memory rehabilitation in Alzheimer's disease: A review of Progress", INT J GERIATR PSYCHIATRY, vol. 16, 2001, pages 794 - 809, XP055850858

## Description

### Technical Field of the Invention:

The present invention relates to the non-medical use of synergistic herbal compositions comprising a first ingredient selected from the extract(s), fraction(s), phytochemical(s) and mixtures thereof derived from *Terminalia chebula* in combination with a second ingredient selected from the oil extract(s)/fraction(s) derived from *Boswellia serrata* gum resin, after removing boswellic acids, for improving at least one cognitive function selected from memory, intelligence, learning, retention, recall information/communication, focus, concentration, attention, perception, reasoning, problem solving, decision making and mental fatigue.

### Background of the invention

Cognition is the process of acquiring knowledge and understanding through thought, experience and the senses. Age-associated cognitive deficit has been well documented in the scientific literature. Cognitive abilities, such as memory, learning, recall information/communication, focus, concentration, reasoning, problem solving, processing speed and decision making etc., decline gradually overtime. Acetylcholine plays pivotal role in the cognitive function. Acetylcholinesterase, also known as AChE, is the primary cholinesterase in the body. Cholinesterase inhibitors (also called acetylcholinesterase inhibitors) are a group of medicines that block the normal breakdown of acetylcholine. Acetylcholine is the main neurotransmitter found in the body and has functions in both peripheral nervous system and central nervous system.

Thus, there is a continuous need in the art to provide potent alternative treatments comprising highly effective herbal extracts for improving brain health or cognitive functions. In this context, there are ample patent publications which are discussed herein below.

Patent publication WO2018087782A1, discloses a synergistic composition containing *Bacopa monnieri* and *Terminalia chebula* for improving brain health, memory/mental condition, enhancing brain/mental functions such as cognition, memory, learning, retention, communication and for treating neurodegenerative diseases/disorders such as Alzheimer's disease, Parkinson's disease etc.

Another patent publication US8962042B2, discloses a method for treating apraxia, autism, speech impairments, traumatic brain injury, seizure disorders, epilepsy, global delays, or ADHD by using a composition containing Gymnema sylvestre, Commiphora mukul, Curcuma longa, Camellia sinensis, Emblica officinalis, and Terminalia chebula.

Patent publication WO2011114350A2, discloses non-acidic extract/fraction selected from Boswellia low polar gum resin extract fraction (BLPRE), Boswellia volatile oil fraction (BVOIL) and Boswellia oil fraction (BOIL) derived from the gum resin of Boswellia species for improving mental condition/brain health; enhancing brain functions such as cognition, memory, learning, communication; for treating impaired memory, and for preventing, control or treating memory and cognition related disorders/diseases.

Another patent publication WO2010029578A2, discloses combination of Boswellia serrata extract selectively enriched in 3-O-acetyl-1 1-keto-β-boswellic acid (AKBA) and Boswellia serrata non-acidic resin extract (BNRE) for the prevention and treatment of diseases associated with inflammation, cognition, neurological disorders, Alzheimer's disease, collagen synthesis, anti-aging, cholesterol lowering and other metabolic disorders.

The other patent publication WO2015118557A1, discloses a composition comprising *Terminalia chebula, Curcuma longa* and *Boswellia serrata* for preventing or treating inflammation or associated conditions thereof.

US 2017/136076 A1 describes therapeutic compositions comprising herbal extracts and essential oils for smoking and vaporization. "Majoon Bara-e- Nisyaan", TKDL, 1 January 1974, XP003029404 discloses a memory enhancing composition comprising *Boswellia serrata* and *Terminalia chebula.* "Arq-e- Gazar Anbari Ba Nuskha Kalan", TKDL, 1 January 1938, XP003031094 describes a brain tonic composition comprising *Boswellia serrata* and *Terminalia chebula.*

However, prior art fails to provide a precise and effective composition specifically for improving cognitive functions such as memory, intelligence, learning, recall information/communication, focus, concentration, reasoning, problem solving, decision making and mental fatigue.

Moreover, there is also a need in the art for better treatment options with minimal side effects thereby making the option safe for human consumption especially when used in long term therapy.

### Objective of the invention

Therefore, the objective of the invention is to provide the use of a synergistic herbal composition comprising a first ingredient selected from the extract(s), fraction(s), phytochemical(s) and mixtures thereof derived from *Terminalia chebula* in combination with a second ingredient selected from the oil extract(s)/fraction(s) derived from *Boswellia serrata* gum resin, after removing boswellic acids, and optionally containing at least one component selected from pharmaceutically or nutraceutically or dietically acceptable excipients, carriers, diluents, biological agent(s) and Nootropic agent(s) for improving at least one cognitive function selected from memory, intelligence, learning, retention, recall information/communication, focus, concentration, attention, perception, reasoning, problem solving, decision making and mental fatigue.

### Summary of the invention

In accordance with the above objective, the present invention provides the non-medical use of synergistic herbal compositions comprising a first ingredient selected from the extract(s), fraction(s), phytochemical(s) and mixtures thereof derived from *Terminalia chebula* in combination with a second ingredient selected from the oil extract(s)/fraction(s) derived from *Boswellia serrata* gum resin, after removing boswellic acids, and optionally containing at least one component selected from pharmaceutically or nutraceutically or dietically acceptable excipients, carriers, diluents, biological agent(s) and Nootropic agent(s) for improving at least one cognitive function selected from memory, intelligence, learning, retention, recall information/communication, focus, concentration, attention, perception, reasoning, problem solving, decision making and mental fatigue,
wherein the first ingredient is a *Terminalia chebula* fruit extract produced using at least one solvent selected from ethanol, methanol, water and mixtures thereof, wherein the second ingredient is produced by extracting *Boswellia serrata* gum resin with at least one solvent selected from ethanol, ethyl acetate, methyl isobutyl ketone and mixtures thereof, and washing with an inorganic base.

### Description of Figures:

**Figure I**: **(A)** Each bar represents the mean ± SD of Target latency (in millisecond, ms); **(B)** distance travelled (cm) by the experimental rats in the acquisition trial in Morris Water Maze. G1 represents the control group of animals supplemented with 10 mL of 0.5% CMC in water; G2, G3 and G4 represent the animal groups receiving 150 mg/kg body weight of B.S-1, T.C-1, and comp-50, respectively. The values (in percentage) above the bars presenting G2-G4 are calculated considering the control (G1) data as 100%. n=6; Intergroup comparison was performed using one-way ANOVA followed by Dunnett's *post hoc* test, *p<0.05 vs. G1.
**Figure II:** The bar graph presents the number of target crossings performed by the rats in the experimental groups in Morris Water Maze-Probe Trial. Bars represent Mean ± SD. The descriptions of G1-G4 are presented above in the Figure I legend. The values (in percentage) above the bars representing G2-G4 are calculated considering the control (G1) data as 100%. Intergroup comparison was performed using one-way ANOVA followed by Dunnett's *post hoc* test, *p<0.05 vs. G1.
**Figure III:** Each bar presents mean ± SD of working memory index (WMI) in T-Maze - Reward Alternation test. The descriptions of G1-G4 are presented in the Figure I legend. The values (in percentage) above the bars representing G2-G4 are calculated considering the control (G1) data as 100%. Intergroup comparison was performed using one-way ANOVA followed by Dunnett's *post hoc* test, **p*<0.05 vs. G1.
**Figure IV:** Each bar presents mean ± SD of serum corticosterone level (nM/L) in the rats of the experimental groups. The descriptions of G1-G4 are presented above in the Figure I legend. The values (in percentage) above the bars representing G2-G4 are calculated considering the control (G1) data as 100%. Intergroup comparison was performed using one-way ANOVA followed by Dunnett's *post hoc* test.
**Figure V:** Each bar presents mean ± SD of total choline concentration (nM/mg protein) in the brain homogenates of the experimental rats. The descriptions of G1-G4 are summarized above in the Figure I legend. The values (in percentage) above the bars presenting G2-G4 are calculated considering the control (G1) data as 100%. Intergroup comparison was performed using one-way ANOVA followed by Dunnett's post hoc test.

### Description of the invention

Ageing is usually associated with the decline in cognitive functions in humans and if untreated, which progresses into more serious conditions, such as dementia and depression, or even Alzheimer's disease. The cholinergic hypothesis is well documented in the literature and suggests that a deficiency of acetylcholine plays a critical role in the genesis of symptoms of degenerative diseases such as age-associated memory loss etc.

In the brain, acetylcholine functions as a neuromodulator and it alters the brain functions. Cholinesterase inhibitors improve cognitive functions and alleviate the symptoms of degenerative disorders through enhancing cortical cholinergic neurotransmission. The ACHE (acetylcholinesterase) inhibitors increase the concentration of acetylcholine and helps to improve cognitive functions such as memory, intelligence, learning, recall information/communication, focus, concentration, reasoning, problem solving, decision making and mental fatigue.

Hence, the inventors of the current application randomly screened a large number of plant extracts and fractions for their acetylcholinesterase (AChE) inhibitory activity, and found that the extracts derived from *Terminalia chebula* and *Boswellia serrata* oil excluding boswellic acids show potent dose dependent acetylcholinesterase inhibitory activities.

Oil extract(s)/fraction(s) derived from the gum resin of *Boswellia serrate,* as used herein refers to the oil extracts/fractions derived from the gum resin of *Boswellia serrate* obtained after removing the boswellic acids, standardized to at least one of epi-α-amyrin, epi-β-amyrin and α-amyrin.

Extract(s), fraction(s), phytochemical(s) and mixtures thereof derived from *Terminalia chebula,* as used herein refers to fruit extract of *Terminalia chebula* standardized to at least one of chebulagic acid or chebulinic acid or ellagic acid or gallic acid or phytochemical(s) and mixtures thereof.

Source of the herbs used in the invention are as follows: -
*1. Terminalia chebula* fruit raw material was collected from Mulapadu village, Krishna district, Andhra Pradesh and it is wild.
*2. Boswellia serrata* gum resin raw material was obtained from Piprani village, Sheopur district, Madhya Pradesh.

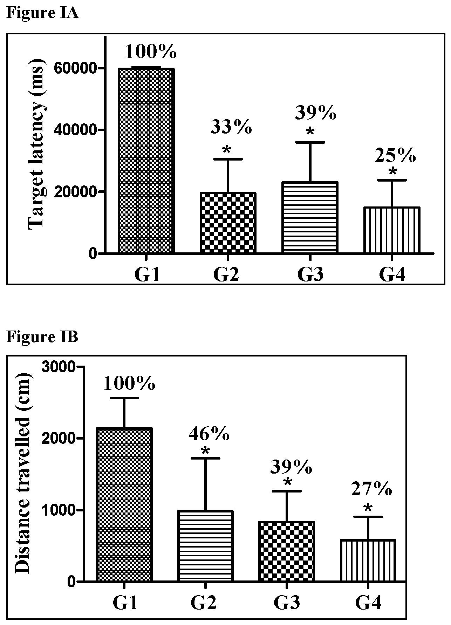

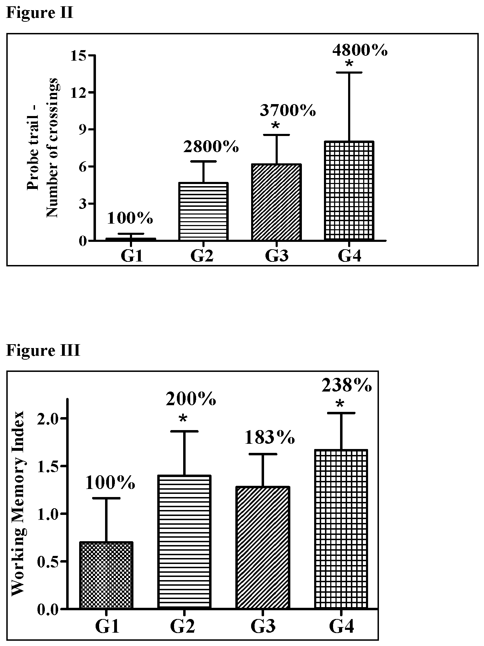

Thus, *Terminalia chebula* fruit raw material was pulverized and the powder was extracted with various solvents such as water, 50% aqueous ethanol, ethanol and 50% aqueous methanol to obtain water extract (T.C-1), 50% aqueous ethanol extract (T.C-2), ethanol extract (T.C-3) and 50% aqueous methanol extract (T.C-4) respectively. The said extracts of Terminalia chebula fruit were standardized to chebulagic acid, chebulinic acid, ellagic acid and gallic acid by analytical HPLC method and the results were summarized in **Table 1.** The chemical structures of ellagic acid and gallic acid are shown in **Figure 1.**

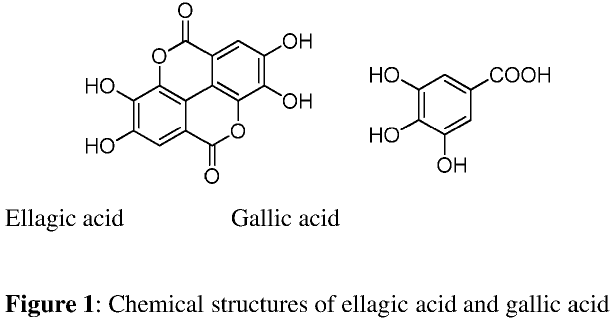

The inventors thought that the *Terminalia chebula* fruit extract enriched to chebulagic acid and chebulinic acid would improve acetylcholinesterase inhibitory activity and show better efficacy in improving cognitive functions.

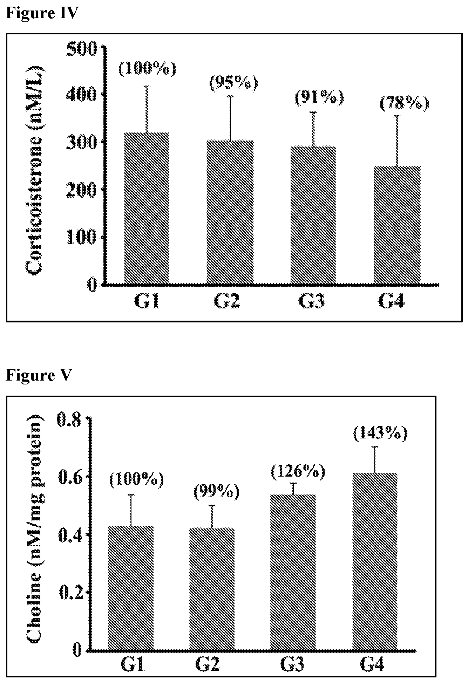

*Terminalia chebula* fruit extract enriched to chebulagic acid and chebulinic acid: Chebulagic acid and chebulinic acid are hydrolysable tannoids known to be responsible for several biological activities such as anti-oxidant, anti-inflammatory and anti-tumor etc. The chemical structures of chebulagic acid and chebulinic acid are shown in **Figure 2.** The extracts enriched in chebulagic acid and chebulinic acid, were produced by eluting the *Terminalia chebula* water extract solution through resin columns. Thus, water extract of *Terminalia chebula is* loaded onto HP-20 resin column and eluted with water, 10% ethanol/water and finally with ethanol. Ethanol fraction was evaporated to get the enriched *Terminalia chebula* extract, which showed 40% by HPLC a sum of chebulagic acid and chebulinic acid. Similarly, with SP-700 and PA-800 resin columns also gave the chebulagic acid and chebulinic acid enriched *Terminalia chebula* extract. The results are tabulated in **Table 2.**

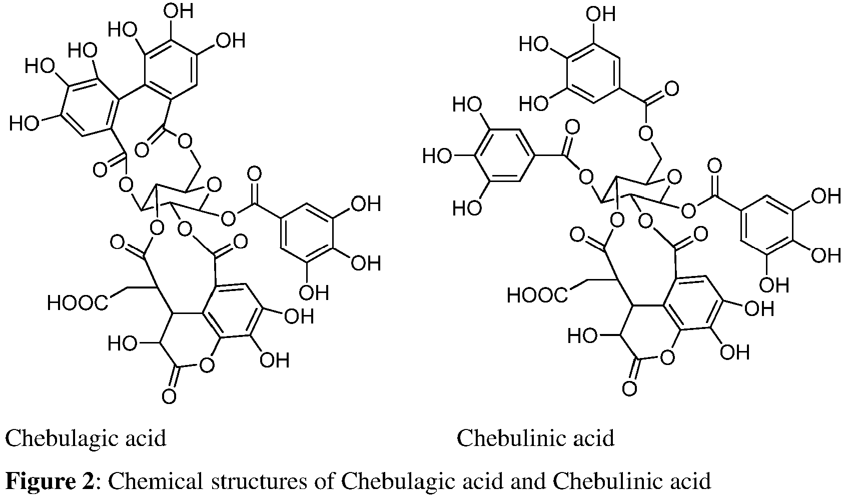

Chebulagic acid and chebulinic acid enriched *Terminalia chebula* fruit extract was evaluated for its acetylcholinesterase inhibitory activity and found that the enriched extract (T.C-5) showed unexpectedly better efficacy than the water extract (T.C-1) as shown below.

| Terminalia chebula extracts | Dose (µg/mL) | % Inhibition of ACHE production |
|---|---|---|
| *Terminalia chebula* water extract (T.C-1) | 40 | 27.09 |
| *Terminalia chebula* extract enriched to chebulagic acid and chebulinic acid (T.C-5) | 40 | 35.22 |

For developing new herbal ingredients for improving cognition and memory functions, *Boswellia serrata* extracts have been screened for their Acetyl cholinesterase inhibitory activity. It was found that the non-acidic extracts or fractions derived from *Boswellia serrata* gum resin showed potent inhibition of acetylcholinesterase, when compared to the inhibition shown by Boswellia serrata extract containing boswellic acids. For example, *Boswellia serrata* extract containing boswellic acids at 50 µg/mL showed 17.09% acetylcholinesterase inhibition, whereas Boswellia serrata oil after removing boswellic acids at 50 µg/mL showed 29.93% acetylcholinesterase inhibitory activity.

*Boswellia serrata* gum resin was extracted with various solvents such as ethanol, ethyl acetate and methyl isobutyl ketone and the acidic compounds were selectively removed by washing with inorganic bases such as sodium carbonate, potassium carbonate and potassium hydroxide to obtain the *Boswellia serrata* gum resin oil.

Thus, *Boswellia serrata* gum resin was extracted with ethanol and basified with aqueous sodium carbonate solution. After evaporation of ethanol, the mixture was extracted with ethyl acetate. Ethyl acetate layer was evaporated and volatile compounds removed to obtain *Boswellia serrata* gum resin oil (B.S-1). Alternatively, *Boswellia serrata* gum resin was extracted with ethyl acetate and the layer was washed with aqueous potassium carbonate solution to remove acidic boswellic acids. Ethyl acetate layer was evaporated and volatile compounds removed to obtain *Boswellia serrata* gum resin oil (B.S-2). Similarly, *Boswellia serrata* gum resin was extracted with methyl isobutyl ketone and the layer was washed with aqueous potassium hydroxide solution to remove boswellic acids. MIBK layer was evaporated and volatile compounds removed to obtain *Boswellia serrata* gum resin oil (B.S-3). The said oil fractions of *Boswellia serrata* gum resin were standardized to total amyrins by analytical HPLC method and the results were summarized in Table 3. Amyrins consists of epi-α-amyrin (3α-Hydroxy-urs-12-en-3-ol), epi-β-amyrin (3α-Hydroxy-olean-12-en-3-ol) and α-amyrin (3β-Hydroxy-urs-12-en-3-ol), and their chemical structures are shown in Figure (3). In addition, the oil fractions also contain serratol, a diterpene compound.

The relevant methods for the preparation of oil extracts/fractions are disclosed by the present inventors in granted patent US8551496B2.

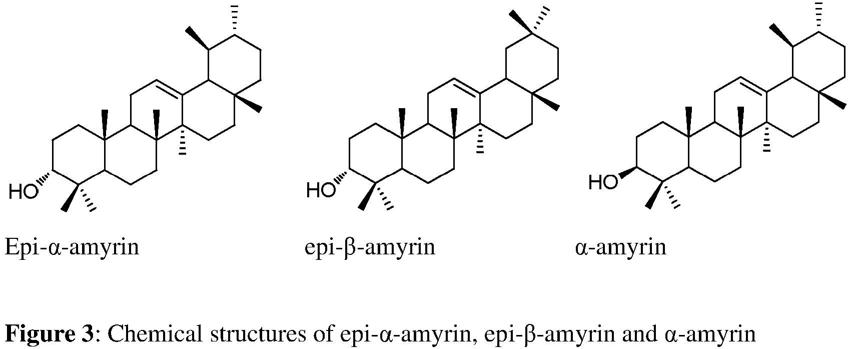

The composition-1 to composition-49 were prepared by combining randomly a first ingredient selected from the extract derived from *Terminalia chebula* fruit with a second ingredient selected from the oil extract(s)/fraction(s) derived from the gum resin of *Boswellia serrata* at different ratios. The compositions so obtained (compositions-1 to 49) were evaluated for acetylcholinesterase (AChE) inhibitory activity in comparison with the corresponding individual ingredients. The data from *in vitro* AChE assay showed that these compositions unexpectedly showed better efficacy in inhibiting acetylcholinesterase when compared to their corresponding individual ingredients suggesting that the individual extracts derived from *Terminalia chebula* have the tendency to exhibit synergism when combined with the oil extract(s)/fraction(s) derived from *Boswellia serrata.*

For example, *Terminalia chebula* water extract (T.C-1) at 40 µg/mL and *Boswellia serrata* oil (B.S-1) at 10 µg/mL showed 27.09% and 10.63% acetylcholinesterase inhibitory respectively. The composition-1 containing these two extracts at 4:1 ratio showed 49.61% acetyl cholinesterase inhibitory activity at 50 µg/mL concentration, which is significantly better than the additive effect (27.09% + 10.63% = 37.71%) from these two ingredients, suggesting synergistic effect between *Terminalia chebula* water extract (T.C-1) and *Boswellia serrata* oil (B.S-1) in inhibiting acetyl cholinesterase. The compositions-2 to 7 obtained when combining these two ingredients at ratios, 3:1, 2:1, 1:1, 1:2, 1:3 and 1:4 respectively also showed synergism in acetylcholinesterase inhibition, when compared to the inhibitions shown by their corresponding individual ingredients as summarized in **Table-4.**

Similarly, the compositions-8 to 14 containing *Terminalia chebula* 50% aq ethanol extract (T.C-2) and *Boswellia serrata* oil (B.S-1); compositions-15 to 21 containing *Terminalia chebula* ethanol extract (T.C-3) and *Boswellia serrata* oil (B.S-1); compositions-22 to 28 containing Terminalia chebula 50% aq methanol extract (T.C-4) and *Boswellia serrata* oil (B.S-1); compositions-29 to 35 containing enriched *Terminalia chebula* extract (T.C-5) and *Boswellia serrata* oil (B.S-1); compositions-36 to 42 containing *Terminalia chebula* water extract (T.C-1) and *Boswellia serrata* oil (B.S-2) and compositions-43 to 49 containing *Terminalia chebula* water extract (T.C-1) and *Boswellia serrata* oil (B.S-3) also showed synergism in acetylcholinesterase inhibition, when compared to the inhibitions shown by their corresponding individual ingredients as summarized in **Tables 5-9.**

### Inhibition of ROS production

Brain is one of the most exquisite organs in the body with high metabolic demands. It maintains body homeostasis by integration and regulation of several central and peripheral signals and requires a tight regulation of the surrounding environment. The tight regulation is exerted by neurovascular unit (NVU) that comprises of different cell types like endothelial cells, neurons, astrocytes, pericytes and equipped with powerful antioxidant defense systems that include reduced glutathione (GSH), superoxide dismutase, catalase etc. Endothelial cells are rich in mitochondria and nicotinamide adenine dinucleotide phosphate (NADPH) oxidases, the two major sources of reactive oxygen species (ROS) production. It is widely known that cellular homeostasis depends on the ROS levels. Physiological ROS levels play important roles in cerebral vasculature as vasodilators, controlling the vascular tone as well as acting as essential signaling molecules for the proper formation of learning and memory. A failure to maintain proper balance between ROS production and their neutralization through the anti-oxidant systems causes the disruption of NVU and brain homeostasis. Excess ROS production has been linked to impaired learning and memory processes through their toxic effect on the neuronal circuitry necessary for memory formation, a predisposing factor for the neurodegenerative conditions.

Hence, the inventors of the present invention further tested said synergistic compositions comprising *Terminalia chebula* extracts and *Boswellia serrata* oil fraction for their antioxidant potential and surprisingly found that these compositions showed superior inhibition of Reactive Oxygen Species (ROS and NOX), when compared to the efficacy shown by their individual ingredients.

For example, the ROS inhibitory activities of *Terminalia chebula* water extract (T.C-1) at 4 µg/mL and *Boswellia serrata* oil (B.S-1) at 1 µg/mL were 32.58% and 0.11% respectively. The composition-1 containing these two extracts at 4:1 ratio showed 40.76% ROS inhibitory activity at 5 µg/mL concentration, which is significantly better than the additive effect (32.58% + 0.11% = 32.69%) from these two ingredients, suggesting synergistic effect between *Terminalia chebula* water extract (T.C-1) and *Boswellia serrata* oil (B.S-1) in inhibiting ROS. The compositions-2 to 7 containing these two ingredients at ratios, 3:1, 2:1, 1:1, 1:2, 1:3 and 1:4 respectively also showed synergism in inhibiting ROS, when compared to the inhibitions shown by their corresponding individual ingredients as summarized in **Table-10.**

Similarly, the compositions 8-49 containing *Terminalia chebula* extracts and *Boswellia serrata* oil fractions at various ratios also showed synergism in ROS inhibition, when compared to the inhibitions shown by their corresponding individual ingredients as summarized in **Tables 11-14.**

### Inhibition of NADPH oxidase (NOX)

Oxidative damage caused by excessive reactive oxygen species (ROS) production promotes cognitive impairment through neurodegeneration. NADPH oxidase is a membrane-bound enzyme complex. Its activation promotes superoxide radical production in the mitochondria and microsomal compartments. Therefore, inhibition of NADPH oxidase (NOX) activity is important to reduce neurodegeneration and to improve neuronal functions that include learning, memory, and other executive functions of the brain.

For example, the NOX inhibitory activity of *Terminalia chebula* water extract (T.C-1) at 400 µg/mL and *Boswellia serrata* oil (B.S-1) at 100 µg/mL were 67.37% and 3.29% respectively. The composition-1 containing these two extracts at 4:1 ratio showed 85.44% NOX inhibitory activity at 500 µg/mL concentration, which is significantly better than the additive effect (67.37% + 3.29% = 70.66%) from these two ingredients, suggesting synergistic effect between *Terminalia chebula* water extract (T.C-1) and *Boswellia serrata* oil (B.S-1) in inhibiting NOX. The compositions-2 to 7 that were obtained when combining these two ingredients at ratios, 3:1, 2:1, 1:1, 1:2, 1:3 and 1:4 respectively also showed synergism in acetylcholinesterase inhibition, when compared to the inhibitions shown by their corresponding individual ingredients as summarized in **Table-15.**

Similarly, the compositions 8-49 containing *Terminalia chebula* extracts and *Boswellia serrata* oil fractions at various ratios also showed synergism in NOX inhibition, when compared to the inhibitions shown by their corresponding individual ingredients as summarized in **Tables 16-21.**

Hence, these compositions (composition 1-49) unexpectedly showed better efficacy to inhibit Acetylcholinesterase, inhibition of ROS and inhibition of NOX when compared to their corresponding individual ingredients suggesting that these individual extract(s) and fraction(s) have the tendency to show synergism when combined together.

**Formulations:** The present invention provides the non-medical use of synergistic herbal compositions comprising combination of the first ingredient selected from the extract(s), fraction(s), phytochemical(s) and mixtures thereof derived from *Terminalia chebula* in combination with a second ingredient selected from the oil extract(s)/fraction(s) derived from *Boswellia serrata* gum resin and optionally containing at least one component selected from pharmaceutically or nutraceutically or dietically acceptable excipients, carriers and diluents.

The compositions comprising combination of the first ingredient selected from the extract(s), fraction(s), phytochemical(s) and mixtures thereof derived from *Terminalia chebula* in combination with a second ingredient selected from the oil extract(s)/fraction(s) derived from *Boswellia serrata* gum resin, after removing boswellic acids; and optionally containing at least one component selected from pharmaceutically or nutraceutically or dietically acceptable excipients, carriers and diluents; are used for improving cognitive functions selected from memory, intelligence, learning, recall information/communication, focus, concentration, reasoning, problem solving, decision making and mental fatigue; wherein the pharmaceutically or nutraceutically or dietically acceptable excipients, carriers and diluents are selected from monosaccharide's such as glucose, dextrose, fructose, galactose etc.; Disaccharides such as sucrose, maltose, lactose, lactulose, trehalose cellobiose, chitobiose etc.; Polycarbohydrates such as Starch and modified starch such as Sodium starch glycolate, pre gelatinized starch, soluble starch, and other modified starches such as Ultrasperse A & Ultra-tex 4; Dextrins that are produced by hydrolysis of starch or glycogen such as yellow dextrin, white dextrin, Maltodextrin etc.; Polyhydric alcohols or sugar alcohols such as Sorbitol, mannitol, inositol, xylitol, isomalt etc.; cellulose based derivatives such as microcrystalline cellulose, hydroxy propyl methyl cellulose, hydroxy ethyl cellulose etc.; silicates such as neusilin, veegum, Talc, colloidal silicon dioxide etc.; metallic stearates such as calcium stearate, magnesium stearate, zinc Stearate etc.; Organic acids such as citric acid, tartaric acid, malic acid, succinic acid, lactic acid, L-ascorbic acid etc.; Fatty acid esters and esters of poly sorbate, natural gums such as acacia, carrageenan, Guar gum, Xanthan gum etc.; vitamin B group, nicotinamide, calcium pantothenate, amino acids, proteins such as casein, gelatin, pectin, agar; organic metal salts such as sodium chloride, calcium chloride, dicalcium phosphate, zinc Sulphate, zinc chloride etc.; Natural pigments, flavors, Class I & Class II preservatives and aqueous, alcoholic, hydro-alcoholic, organic solutions of above listed ingredients alone or in combination.

**Evaluation of the composition-50 for Nootropic activity (Learning and Memory enhancement and stress) in Sprague-Dawley rats:** The *in vitro* efficacy shown by these compositions were further validated through an *in vivo* experiment in Sprague Dawley rat model. The efficacy of composition-50 containing *Terminalia chebula* water extract (T.C-1) and *Boswellia serrata* oil (B.S-1) in the ratio of 4:1 on memory enhancement was assessed in Morris Water Maze (MWT) and T-Maze in comparison with the individual ingredients.

The target latency and the travelled distance were significantly improved in the rats supplemented with the test substances, when compared to the control rats. In addition, these groups supplemented with the test substances also showed increased number of target crossings in comparison with the control. Together, these observations indicate that the supplementation of *Boswellia serrata* oil (B.S-1), *Terminalia chebula* water extract (T.C-1) and composition-50 improved spatial learning in the rats. Spatial learning refers to the process through which animals encode information about their environment to facilitate navigation through space and recall the location of motivationally relevant stimuli. In particular, the composition composition-50 supplemented rats showed better improvements in spatial learning and memory than the individual ingredients of the formula. The working memory index (WMI) was also increased in the herbal supplemented groups compared control rats; and the composition supplemented rats showed better improvement than the individual extracts. These data suggest that the ingredients and their composition are effective in improving short term working memory of the rats. Further; the decreased serum corticosterone level in the composition supplemented rats suggests that the inventive composition is effective in reducing stress in the experimental rats. Also, increased choline level in the brain indicates improved brain function, including better short term and long term memory and information processing in the composition supplemented rats.

**Figures IA and IB** show the measures of spatial learning activity of the ingredients, *Boswellia serrata* oil (B.S-1) and *Terminalia chebula* water extract (T.C-1) and their composition-50 (Comp-50) supplemented rats. Figure-IA depicts, the B.S-1 and T.C-1 supplemented rats took 33% and 39%, respectively of the time taken by the control rats; while the composition (comp-50) supplemented rats took 25% of the time taken by control rats. Also, the B.S-1 and T.C-1 rats showed 54% and 61% improvements, respectively; while the composition (comp-50) supplemented showed 73% improvement in comparison with the control rats in travelled distance to achieve the target (Figure IB). In target latency and distance travelled measures, the composition (comp-50) supplemented rats performed significantly better than the individual ingredients, supplemented at the same dose. These observations suggest synergistic improvements of spatial learning in the composition supplemented rats compared to its individual components.

**Figure-II** shows the spatial memory efficacy of the ingredients and their composition. In a stipulated time, the B.S-1 and T.C-1 supplemented rats performed 28 and 37 times number of target crossings, respectively; while the composition supplemented rats crossed 48 times than the control rats. The composition (comp-50) supplemented rats performed significantly better than the individual ingredients, supplemented at the same dose. This observation suggests a synergistic improvement of spatial memory in the composition supplemented rats compared to its individual components. Spatial memory is a cognitive process that enables a person to remember different locations as well as spatial relations between objects. This allows one to remember where an object in relation to another object.

**Figure-III** shows the working memory index (WMI) of ingredients and their composition supplemented rats. In comparison with the control rats (100%), the WMI of composition-50 supplemented rats was 238%; while, the B.S-1 and T.C-1 supplemented rats showed 200% and 183%, respectively, at the same dose. The enhanced WMI in the composition-50 rats was significantly better than the two ingredients, suggests a synergistic benefit in increasing the WMI in short term memory in the rats. The working memory index (WMI) measures short term memory and indicates the ability to hold verbal and visual information in mind with sustained mental control.

**Figure-IV** depicts reductions of serum corticosterone levels in the experimental groups of rats. The mean corticosterone levels in B.S-1, T.C-1, and the composition-50 supplemented rats were reduced to 95%, 91%, and 78%, respectively, compared with the control rats (considered as 100%). The decrease in serum corticosterone level in composition-50 supplemented rats was significantly greater than the stand alone ingredients supplemented rats. Corticosterone (CORT) is a major stress hormone secreted from the adrenal cortex by the neuro-endocrine signal generated from pituitary, when an animal is stressed. This observation indicates that the composition is effective in reducing stress.

**Figure-V** presents increase of total choline level in the brain homogenates of the composition supplemented rats, compared with the B.S-1 and T.C-1 supplemented rats. The choline level did not change in B.S-1 group; the T.C-1 supplemented rats showed 26% increase from the control rats. In contrast, composition-50 supplementation yielded 43% increase of choline level in the brain homogenate, compared with the control rats. The increase of choline level in the composition-50 supplemented rats was significantly better than the stand alone ingredients supplemented rats. Increased choline level in the brain indicates improved brain function, including better short term and long term memory and enhanced ability of information processing.

Thus the composition-50 containing Boswellia serrata oil (B.S-1) and Terminalia chebula water extract (T.C-1) in 1:4 ratio showed synergistic efficacy compared to its ingredients in enhancing memory, intelligence, recall information/communication, focus, concentration, reasoning, problem solving, decision making and reducing stress as presented in example 22 and figures I-V. This suggests the possible use of these compositions as a therapeutic option for improving cognitive functions.

The foregoing thus demonstrates that the synergistic herbal compositions of the current invention comprising combination of a first ingredient selected from the extract(s), fraction(s), active compound(s) and phytochemicals or mixtures thereof derived from the plant parts of *Terminalia chebula* and a second ingredient selected from the oil extract(s)/fraction(s) derived from the gum resin of *Boswellia serrata,* after removing boswellic acids, have the potential to inhibit acetylcholinesterase and hence improve cognitive functions such as memory, intelligence, learning, recall information/communication, focus, concentration, reasoning, problem solving, decision making and mental fatigue.

Therefore, the present invention provides the non-medical use of synergistic herbal compositions comprising a first ingredient selected from the extract(s), fraction(s), phytochemical(s) and mixtures thereof derived from *Terminalia chebula* in combination with a second ingredient selected from the oil extract(s)/fraction(s) derived from *Boswellia serrata* gum resin, after removing boswellic acids, for improving at least one cognitive function, selected from memory, intelligence, learning, retention, recall information/communication, focus, concentration, attention, perception, reasoning, problem solving, decision making and mental fatigue.

In an another important embodiment, the synergistic herbal compositions comprise *Terminalia chebula* extract enriched in chebulagic acid and chebulinic acid; and *Boswellia serrata* oil enriched in serratol, epi-α-amyrin, epi-β-amyrin and α-amyrin, excluding boswellic acids.

The synergistic herbal compositions optionally contain at least one component selected from pharmaceutically or nutraceutically or dietically acceptable excipients, carriers, diluents, biological agent(s) and Nootropic agent(s).

The first ingredient is a *Terminalia chebula* fruit extract produced using at least one solvent selected from ethanol, methanol, water and mixtures thereof. The second ingredient is produced by extracting Boswellia serrata gum resin with at least one solvent selected from ethanol, ethyl acetate, methyl isobutyl ketone and mixtures thereof, and washing with an inorganic base.

Described, but not claimed *per se,* is that the plant parts used for preparing the extracts can be selected from leaves, stems, tender stems, tender twigs, aerial parts, whole fruit, fruit rind, seed, gum resin flower heads, root, bark, hardwood or whole plant or mixtures thereof.

In another embodiment, the extracts or fractions are standardized to at least one phytochemical reference marker compound or biological active marker in the extract or fraction.

The present invention provides non-therapeutic methods of improving at least one cognitive function selected from memory, intelligence, learning, retention, recall information/communication, focus, concentration, attention, perception, reasoning, problem solving, decision making and mental fatigue in a human, wherein the method comprises supplementing the human with an effective dose of a synergistic herbal composition described herein.

Described, but not claimed *per se,* are methods of treating brain related diseases/disorders which include but not limited to dementia, dyslexia, aphasia, organic brain syndrome, myasthenia gravis, mild cognitive impairment (NCI), Alzheimer's disease, Parkinson's disease, attention-deficit hyperactivity disorder (ADHD), hypoxia, anoxia, cerebrovascular insufficiency, epilepsy, myoclonus, hypo cholinergic dysfunctions, memory deterioration, functional loss, memory impairment disorders, neurodegenerative disorders, and for controlling blood pressure and blood circulation in the brain in a human, wherein the method comprises supplementing the human with an effective dose of a compositions comprising a first ingredient selected from the extract(s), fraction(s), phytochemical(s) and mixtures thereof derived from *Terminalia chebula* in combination with a second ingredient selected from the oil extract(s)/fraction(s) derived from *Boswellia serrata* gum resin, after removing boswellic acids, and optionally containing at least one component selected from pharmaceutically or nutraceutically or dietically acceptable excipients, carriers, diluents, biological agent(s) and Nootropic agent(s).

The synergistic herbal compositions optionally contain at least one component selected from pharmaceutically or nutraceutically or dietically acceptable excipients, carriers, diluents, biological agent(s) and Nootropic agent(s); wherein the excipients, carriers or diluents are selected from monosaccharide's such as glucose, dextrose, fructose, galactose etc.; Disaccharides such as sucrose, maltose, lactose, lactulose, trehalose cellobiose, chitobiose etc.; Polycarbohydrates such as Starch and modified starch such as Sodium starch glycolate, pre gelatinized starch, soluble starch, and other modified starches such as Ultrasperse A & Ultra-tex 4; Dextrins that are produced by hydrolysis of starch or glycogen such as yellow dextrin, white dextrin, Maltodextrin etc.; Polyhydric alcohols or sugar alcohols such as Sorbitol, mannitol, inositol, xylitol, isomalt etc.; cellulose based derivatives such as microcrystalline cellulose, hydroxy propyl methyl cellulose, hydroxy ethyl cellulose, sodium carboxymethylcellulose (sodium CMC) etc.; silicates such as neusilin, veegum, Talc, colloidal silicon dioxide etc.; metallic stearates such as calcium stearate, magnesium stearate, zinc Stearate etc.; Organic acids such as citric acid, tartaric acid, malic acid, succinic acid, lactic acid, L-ascorbic acid etc.; Fatty acid esters and esters of poly sorbate, natural gums such as acacia, carrageenan, Guar gum, Xanthan gum etc.; vitamin B group, nicotinamide, calcium pantothenate, amino acids, proteins such as casein, gelatin, pectin, agar; organic metal salts such as sodium chloride, calcium chloride, dicalcium phosphate, magnesium hydroxide, zinc Sulphate, zinc chloride etc.; Natural pigments, flavors, Class I & Class II preservatives and aqueous, alcoholic, hydro-alcoholic, organic solutions of above listed ingredients alone or in combination.

In another embodiment, the composition(s) of the present invention may be formulated into a dosage form selected from dry powder form, liquid form, beverage, food product, dietary supplement or any suitable form such as tablet, a capsule or a soft chewable or gummy bear.

In another embodiment of the invention, the composition(s) as disclosed above can be formulated into nutritional/dietary supplements that can be contemplated/made into the dosage form of healthy foods, or food for specified health uses such as solid food like chocolate or nutritional bars, semisolid food like cream or jam, or gel and also beverage and the like, such as refreshing beverage, instant beverage, functional beverages for sports athletes, exercising and muscle building for individuals, lactic acid bacteria beverage, drop, candy, chewing gum, gummy candy, yoghurt, ice cream, pudding, soft adzuki bean jelly, jelly, cookie, tea, soft drink, juice, milk, coffee, cereal, snack bar and the like.

In yet another embodiment, the weight of the first ingredient varies in the range of 10%-90% and the weight of the second ingredient varies in the range of 90% -10% in the composition.

In yet another embodiment, the composition is standardized to at least one *Terminalia chebula* marker selected from gallic acid, ellagic acid, chebulagic acid, chebulinic acid and at least one *Boswellia serrata* gum resin marker selected from epi-α-amyrin, epi-β-amyrin and α-amyrin; wherein the markers in the composition is in the range of 0.1% to 50% by weight of the composition.

In another embodiment, the composition(s) of the present invention can be delivered in the form of controlled release tablets, using controlled release polymer-based coatings by the techniques including nanotechnology, microencapsulation, colloidal carrier systems and other drug delivery systems for obtaining the desired therapeutic benefit.

The presented examples illustrate the invention,

### Examples

### Example 1: Preparation of Terminalia chebula water extract

To Terminalia chebula dried fruit powder (100 g) was added water (400 mL) at rt. The mixture was stirred at 100°C for 2 h and the extract separated by filtration. The extraction process was repeated with water (2 × 300 mL) under similar conditions. The combined extracts were filtered through celite and evaporated under reduced pressure to obtain concentrated extract, which was further dried in vacuum to give the product as a powder (T.C-1; 45 g).

### Example 2: Preparation of Terminalia chebula 50% ethanol extract

To Terminalia chebula dried fruit powder (100 g) was added 50% aqueous ethanol (400 mL) at rt. The mixture was stirred under reflux for 2 h and the residue separated by filtration. The extraction process was repeated with 50% aqueous ethanol (2 × 300 mL) under similar conditions. The combined extracts were filtered through celite and evaporated under reduced pressure to obtain concentrated extract, which was further dried in vacuum to give the product as a powder (T.C-2; 43.5 g).

### Example 3: Preparation of Terminalia chebula ethanol extract

To Terminalia chebula dried fruit powder (100 g) was added ethanol (400 mL) at rt. The mixture was stirred at reflux temperature for 2 h and the extract separated by filtration. The extraction process was repeated with ethanol (2 × 300 mL) under similar conditions. The combined extracts were filtered through celite and evaporated under reduced pressure to obtain concentrate extract, which was further dried in vacuum to give the product as a powder (T.C-3; 43 g).

### Example 4: Preparation of Terminalia chebula 50% methanol extract

To Terminalia chebula dried fruit powder (100 g) was added 50% aqueous methanol (400 mL) at rt. The mixture was stirred at reflux temperature for 2 h and the extract separated by filtration. The extraction process was repeated with 50% aqueous methanol (2 × 400 mL) under similar conditions. The combined extracts were filtered through celite and evaporated under reduced pressure to obtain concentrate extract, which was further dried in vacuum to give the product as a powder (T.C-4; 46 g).

**Table-1: HPLC analysis data of Terminalia chebula extracts**

| **Example No.** | **Solvent for extraction** | **Extract code** | **Product weight** | **HPLC** | | |
|---|---|---|---|---|---|---|
| | | | | **chebulagic acid + chebulinic acid** | **Ellagic acid** | **Gallic acid** |
| 1 | Water | T.C-1 | 45 g | 7.68 % | 1.6 % | 5.5 % |
| 2 | 50% aq ethanol | T.C-2 | 43.5 g | 16.2 % | 1.3 % | 2.0 % |
| 3 | Ethanol | T.C-3 | 43 g | 18.3 % | 2.2 % | 2.3 % |
| 4 | 50% aq methanol | T.C-4 | 46 g | 7.0% | 1.7 % | 3.1 % |

### Example 5: Enrichment of Terminalia chebula extracts using PA-800 resin

1.0 Kg of *Terminalia chebula* raw material was pulverized and passed through 10 # (mesh). The powder was extracted with water (20 L) at 60±5°C for two hours, and the extract solution was filtered on super cell bed. The filtered extract solution (TS: 4.1% by brisk meter) was loaded on 4.6 L of PA-800 resin with flow rate of 3-4 L per hour. After loading the extract, column was eluted sequentially with water (4.6 L), 10% ethanol/water (4.6 L), and finally with ethanol (5.4 L) with same flow. The fractions eluted with each solvent was collected separately and concentrated individually. More specifically, the ethanol fraction was concentrated and dried under vacuum to get the product as a powder (T.C-5; 234 g).

### Example 6: Enrichment of Terminalia chebula extracts using SP-700

1.0 Kg of *Terminalia chebula* raw material was pulverized and passed through 10 # (mesh). The raw material powder was extracted with water (2 × 8.0 L) at 60±5°C for two hours for each extraction. The combined extract solution was filtered on super cell bed. The filtered extract (TS: 3.4% by brisk meter) was loaded on 7.0 L of SP-700 resin, and the column was eluted sequentially with water (12.0 L), 10% ethanol/water (12.0 L), and finally with ethanol (10.0 L). The fraction eluted with each solvent was collected separately and concentrated individually. More specifically, the ethanol fraction was concentrated and dried under vacuum to get the product as a powder (T.C-6; 312 g).

### Example 7: Enrichment of Terminalia chebula extracts using HP-20

1.0 kg of *Terminalia chebula* raw material was pulverized and passed through 10 # (mesh). The powder was extracted with water (18 L) at 70°C for two hours, and the extract solution was filtered on super cell bed. The filtered extract solution (TS: 4.2 % by brisk meter) was loaded on 5 L of HP-20 resin with flow rate of 6-7 L per hour. After loading the extract, column was eluted sequentially with water (5 L), 10% ethanol/water (5 L), and finally with ethanol (7 L) using same flow rate. The fraction eluted with each percentage of solvent was collected separately and concentrated individually. More specifically, the ethanol fraction was concentrated and dried under vacuum to get the product as a powder (T.C-7; 217 g).

**Table-2: HPLC analysis data of enriched Terminalia chebula extracts**

| **Example No.** | **Extract code** | **Product weight** | **HPLC assay (Chebulagic acid + Chebulinic acid)** |
|---|---|---|---|
| 4 | T.C-5 | 234 g | 40.0% |
| 5 | T.C-6 | 312 g | 31.8% |
| 6 | T.C-7 | 217 g | 46.9% |

### Example 8: Preparation of Boswellia serrata oil by ethanol extraction

B. serrata resin (100 g) was extracted with 85% aq ethanol (300 mL) under stirring at reflux temperature for 3 h. The spent gum residue was separated by filtration and extracted again using same extraction conditions. The combined extract solution was filtered through hyflow gel. The bed was washed with 85% aq ethanol (25 mL). The solution pH was adjusted to 8.8-9.0 with 20% aqueous sodium carbonate solution under stirring and settled at rt for 30 min. The extract solution was filtered and concentrated under vacuum to 100 mL volume. Water (100 mL) was added and stirred at rt for 10 min. EtOAc (100 mL) was added to the mixture and maintained under stirring for 10 min and settled for 30 min. The aqueous layer was separated and the ethyl acetate layer was evaporated under reduced pressure to give the oily compound. The volatile compounds were then removed from the oil under reduced pressure and high temperature to obtain Boswellia oil product (B.S-1; 20.5 g).

### Example 9: Preparation of Boswellia serrata oil by ethyl acetate extraction

To B. serrata resin (100 g) was added ethyl acetate (400 mL) at rt. The mixture was stirred at reflux temperature for 2 h and the extract filtered. The extraction process was repeated with ethyl acetate (200 mL). The combined extract was filtered through celite bed and distilled under reduced pressure to 250 mL volume. To this ethyl acetate extract concentrate, 2% potassium carbonate (250 mL) was added and stirred for 30 min. After settling for 30 min, the aqueous layer was separated using separator funnel. This process was repeated twice with 2% potassium carbonate (2 × 250 mL). The ethyl acetate layer was evaporated under reduced pressure to give Boswellia serrata oil. The volatile compounds were then removed from the oil under reduced pressure and high temperature to obtain Boswellia oil (B.S-2; 31 g).

### Example 10: Preparation of Boswellia serrata oil by MIBK extraction

To B. serrata resin (100 g) was dispersed in methyl isobutyl ketone (MIBK; 200 mL) and stirred at 80-85°C for 1 h. The extract was separated and the residue extracted again with MIBK (100 mL) under similar conditions. The combined MIBK extracts was filtered and treated with 2% aqueous sodium hydroxide solution (300 mL) under stirring for 30 min. After settling for 30 min, aqueous layer was separated using separator funnel. This process was repeated with 2% aqueous sodium hydroxide (100 mL). The combined MIBK layer was evaporated under reduced pressure to give thick paste. This was further concentrated under vacuum and heating to obtain the product as oil (B.S-3; 20 g).

**Table-3: HPLC analysis data of Boswellia serrata oil**

| **Example No.** | **Extract code** | **Solvent for extraction** | **Weight of the product** | **HPLC of total amyrins** (epi-α-amyrin, epi-β-amyrin and α-amyrin) |
|---|---|---|---|---|
| 8 | B.S-1 | 85% aq ethanol | 20.5 g | 9.7% |
| 9 | B.S-2 | Ethyl acetate | 31.0 g | 12.1% |
| 10 | B.S-3 | Methyl isobutyl ketone (MIBK) | 20.0 g | 8.5% |

### Example 11: Preparation of compositions containing Terminalia chebula water extract and Boswellia serrata oil by ethanol extraction.

Composition-1 (C-1): The composition-1 was prepared by combining Terminalia chebula water extract (T.C-1) and Boswellia serrata oil (B.S-1) in the ratio of 4:1. Composition-2 (C-2): The composition-2 was prepared by combining Terminalia chebula water extract (T.C-1) and Boswellia serrata oil (B.S-1) in the ratio of 3:1. Composition-3 (C-3): The composition-3 was prepared by combining Terminalia chebula water extract (T.C-1) and Boswellia serrata oil (B.S-1) in the ratio of 2:1. Composition-4 (C-4): The composition-4 was prepared by combining Terminalia chebula water extract (T.C-1) and Boswellia serrata oil (B.S-1) in the ratio of 1:1. Composition-5 (C-5): The composition-5 was prepared by combining Terminalia chebula water extract (T.C-1) and Boswellia serrata oil (B.S-1) in the ratio of 1:2. Composition-6 (C-6): The composition-6 was prepared by combining Terminalia chebula water extract (T.C-1) and Boswellia serrata oil (B.S-1) in the ratio of 1:3. Composition-7 (C-7): The composition-7 was prepared by combining Terminalia chebula water extract (T.C-1) and Boswellia serrata oil (B.S-1) in the ratio of 1:4.

### Example 12: Preparation of compositions containing Terminalia chebula 50% aq ethanol extract and Boswellia serrata oil by ethanol extraction.

Composition-8 (C-8): The composition-8 was prepared by combining Terminalia chebula 50% aqueous ethanol extract (T.C-2) and Boswellia serrata oil (B.S-1) in the ratio of 4:1.

Composition-9 (C-9): The composition-9 was prepared by combining Terminalia chebula 50% aqueous ethanol extract (T.C-2) and Boswellia serrata oil (B.S-1) in the ratio of 3:1.

Composition-10 (C-10): The composition-10 was prepared by combining Terminalia chebula 50% aqueous ethanol extract (T.C-2) and Boswellia serrata oil (B.S-1) in the ratio of 2:1.

Composition-11 (C-11): The composition-11 was prepared by combining Terminalia chebula 50% aqueous ethanol extract (T.C-2) and Boswellia serrata oil (B.S-1) in the ratio of 1:1.

Composition-12 (C-12): The composition-12 was prepared by combining Terminalia chebula 50% aqueous ethanol extract (T.C-2) and Boswellia serrata oil (B.S-1) in the ratio of 1:2.

Composition-13 (C-13): The composition-13 was prepared by combining Terminalia chebula 50% aqueous ethanol extract (T.C-2) and Boswellia serrata oil (B.S-1) in the ratio of 1:3.

Composition-14 (C-14): The composition-14 was prepared by combining Terminalia chebula 50% aqueous ethanol extract (T.C-2) and Boswellia serrata oil (B.S-1) in the ratio of 1:4.

### Example 13: Preparation of compositions containing Terminalia chebula ethanol extract and Boswellia serrata oil by ethanol extraction.

Composition-15 (C-15): The composition-15 was prepared by combining Terminalia chebula ethanol extract (T.C-3) and Boswellia serrata oil (B.S-1) in the ratio of 4:1.

Composition-16 (C-16): The composition-16 was prepared by combining Terminalia chebula ethanol extract (T.C-3) and Boswellia serrata oil (B.S-1) in the ratio of 3:1.

Composition-17 (C-17): The composition-17 was prepared by combining Terminalia chebula ethanol extract (T.C-3) and Boswellia serrata oil (B.S-1) in the ratio of 2:1.

Composition-18 (C-18): The composition-18 was prepared by combining Terminalia chebula ethanol extract (T.C-3) and Boswellia serrata oil (B.S-1) in the ratio of 1:1.

Composition-19 (C-19): The composition-19 was prepared by combining Terminalia chebula ethanol extract (T.C-3) and Boswellia serrata oil (B.S-1) in the ratio of 1:4.

Composition-20 (C-20): The composition-20 was prepared by combining Terminalia chebula ethanol extract (T.C-3) and Boswellia serrata oil (B.S-1) in the ratio of 1:3.

Composition-21 (C-21): The composition-21 was prepared by combining Terminalia chebula ethanol extract (T.C-3) and Boswellia serrata oil (B.S-1) in the ratio of 1:4.

### Example 14: Preparation of compositions containing Terminalia chebula 50% aq methanol extract and Boswellia serrata oil by ethanol extraction.

Composition-22 (C-22): The composition-22 was prepared by combining Terminalia chebula 50% aqueous methanol extract (T.C-4) and Boswellia serrata oil (B.S-1) in the ratio of 4:1.

Composition-23 (C-23): The composition-23 was prepared by combining Terminalia chebula 50% aqueous methanol extract (T.C-4) and Boswellia serrata oil (B.S-1) in the ratio of 3:1. Composition-24 (C-24): The composition-24 was prepared by combining Terminalia chebula 50% aqueous methanol extract (T.C-4) and Boswellia serrata oil (B.S-1) in the ratio of 2:1. Composition-25 (C-25): The composition-25 was prepared by combining Terminalia chebula 50% aqueous methanol extract (T.C-4) and Boswellia serrata oil (B.S-1) in the ratio of 1:1. Composition-26 (C-26): The composition-26 was prepared by combining Terminalia chebula 50% aqueous methanol extract (T.C-4) and Boswellia serrata oil (B.S-1) in the ratio of 1:2. Composition-27 (C-27): The composition-27 was prepared by combining Terminalia chebula 50% aqueous methanol extract (T.C-4) and Boswellia serrata oil (B.S-1) in the ratio of 1:3. Composition-28 (C-28): The composition-28 was prepared by combining Terminalia chebula 50% aqueous methanol extract (T.C-4) and Boswellia serrata oil (B.S-1) in the ratio of 1:4.

### Example 15: Preparation of compositions containing enriched Terminalia chebula extract and Boswellia serrata oil by ethanol extraction.

Composition-29 (C-29): The composition-29 was prepared by combining enriched Terminalia chebula extract (T.C-5) and Boswellia serrata oil (B.S-1) in the ratio of 4:1.

Composition-30 (C-30): The composition-30 was prepared by combining enriched Terminalia chebula extract (T.C-5) and Boswellia serrata oil (B.S-1) in the ratio of 3:1.

Composition-31 (C-31): The composition-31 was prepared by combining enriched Terminalia chebula extract (T.C-5) and Boswellia serrata oil (B.S-1) in the ratio of 2:1.

Composition-32 (C-32): The composition-32 was prepared by combining enriched Terminalia chebula extract (T.C-5) and Boswellia serrata oil (B.S-1) in the ratio of 1:1.

Composition-33 (C-33): The composition-33 was prepared by combining enriched Terminalia chebula extract (T.C-5) and Boswellia serrata oil (B.S-1) in the ratio of 1:2.

Composition-34 (C-34): The composition-34 was prepared by combining enriched Terminalia chebula extract (T.C-5) and Boswellia serrata oil (B.S-1) in the ratio of 1:3.

Composition-35 (C-35): The composition-35 was prepared by combining enriched Terminalia chebula extract (T.C-5) and Boswellia serrata oil (B.S-1) in the ratio of 1:4.

### Example 16: Preparation of compositions containing Terminalia chebula water extract and Boswellia serrata oil by ethyl acetate extraction.

Composition-36 (C-36): The composition-36 was prepared by combining Terminalia chebula water extract (T.C-1) and Boswellia serrata oil (B.S-2) in the ratio of 4:1.

Composition-37 (C-37): The composition-37 was prepared by combining Terminalia chebula water extract (T.C-1) and Boswellia serrata oil (B.S-2) in the ratio of 3:1.

Composition-38 (C-38): The composition-38 was prepared by combining Terminalia chebula water extract (T.C-1) and Boswellia serrata oil (B.S-2) in the ratio of 2:1.

Composition-39 (C-39): The composition-39 was prepared by combining Terminalia chebula water extract (T.C-1) and Boswellia serrata oil (B.S-2) in the ratio of 1:1.

Composition-40 (C-40): The composition-40 was prepared by combining Terminalia chebula water extract (T.C-1) and Boswellia serrata oil (B.S-2) in the ratio of 1:2.

Composition-41 (C-41): The composition-41 was prepared by combining Terminalia chebula water extract (T.C-1) and Boswellia serrata oil (B.S-2) in the ratio of 1:3.

Composition-42 (C-42): The composition-42 was prepared by combining Terminalia chebula water extract (T.C-1) and Boswellia serrata oil (B.S-2) in the ratio of 1:4.

### Example 17: Preparation of compositions containing Terminalia chebula water extract and Boswellia serrata oil by MIBK extraction.

Composition-43 (C-43): The composition-43 was prepared by combining Terminalia chebula water extract (T.C-1) and Boswellia serrata oil (B.S-3) in the ratio of 4:1.

Composition-44 (C-44): The composition-44 was prepared by combining Terminalia chebula water extract (T.C-1) and Boswellia serrata oil (B.S-3) in the ratio of 3:1.

Composition-45 (C-45): The composition-45 was prepared by combining Terminalia chebula water extract (T.C-1) and Boswellia serrata oil (B.S-3) in the ratio of 2:1.

Composition-46 (C-46): The composition-46 was prepared by combining Terminalia chebula water extract (T.C-1) and Boswellia serrata oil (B.S-3) in the ratio of 1:1.

Composition-47 (C-47): The composition-47 was prepared by combining Terminalia chebula water extract (T.C-1) and Boswellia serrata oil (B.S-3) in the ratio of 1:2.

Composition-48 (C-48): The composition-48 was prepared by combining Terminalia chebula water extract (T.C-1) and Boswellia serrata oil (B.S-3) in the ratio of 1:3.

Composition-49 (C-49): The composition-49 was prepared by combining Terminalia chebula water extract (T.C-1) and Boswellia serrata oil (B.S-3) in the ratio of 1:4.

### Example 18: Formulation of the composition.

Composition-50 (C-50): To 72 ml of water was added 72g of T.C-1 under continuous stirring using Remi stirrer at 200-400 rpm. To this extract solution was added 3.0g of Ultrasperse A under continuous stirring. After complete addition, continue stirring for further 20 min. To the above mixture add 1.25g of sodium carboxy methyl cellulose, 0.75g of magnesium hydroxide and 3.0g of micro crystalline cellulose and continue stirring for 20min.

Separately 18g of B.S-1 was dissolved in 14 ml of ethanol and the ethanolic solution of B.S-1 added to the above mixture and continued the stirring for about 30 min. The mixture was transferred into clean tray sheet and dried under vacuum at 60-70°C to get dry flakes. The flakes were pulverized by adding 2.0 g of syloid and passed through suitable sieve to obtain composition-50 as a free flowing powder.

### Example 19: General procedure for Acetylcholinesterase inhibitory activity

Acetylcholinesterase activity was measured using the substrate acetylthiocholine iodide, which converts to thiocholine. The reaction of thiocholine with the chromogenic substrate Dithionitrobenzoic acid (DTNB) leads to the formation of a yellow anion of 2-thio- 2-Nitrobenzoic acid, which absorbs strongly at 412 nm.

The AChE assay was performed by the method of Ellman et. al, with minor modifications, using acetylthiocholine iodide as a substrate (Lee J. H., et. al. Arch Pharm Res 2004, 27(1): 53-56). Ellmans reaction mixture contains, 1.0 mM acetylthiocholine iodide and 0.5 mM 5, 5'-dithio-bis-(2-nitrobenzoic acid) in a 50 mM sodium phosphate buffer (pH 8.0). The assay mixture contained 50 µ1 of 50 mM phosphate buffer at pH - 8.0, 30 µl of test substance at various concentrations and 20 µl of (100 mU/mL) enzyme. For blanks, the enzyme was replaced with phosphate buffer. The reaction mixture was mixed thoroughly, 100 µl of Ellman's reagent was added and incubated at room temperature for 10 min. The absorbance was measured at 412nm using microplate reader. The percentage inhibition of enzyme activity was calculated by comparing OD's of tests wells with that of control wells using the following formula. Calculations: % inhibition = [(control-sample)/control] x 100. The results of Acetylcholinesterase inhibitory activity of the individual extracts and their compositions are summarized in **Tables 4-9.**

**Table-4: Acetyl cholinesterase inhibitory activities of the compositions containing Terminalia chebula water extract (T.C-1) and Boswellia serrata oil (B.S-1).**

| Comp # | T.C-1 | | B.S-1 | | Ratio | Comp Dose µg/mL | % Inhibition of ACHE production | |
|---|---|---|---|---|---|---|---|---|
| | µg/mL | % Inhibition | µg/mL | % Inhibition | | | Additive (Calculated) | Observed |
| C-1 | 40 | 27.09 | 10 | 10.63 | 4:1 | 50 | 37.71 | 49.61 |
| C-2 | 37.5 | 25.40 | 12.5 | 13.28 | 3:1 | 50 | 38.68 | 44.98 |
| C-3 | 33.33 | 22.57 | 16.67 | 17.71 | 2:1 | 50 | 40.28 | 50.49 |
| C-4 | 25 | 16.93 | 25 | 26.57 | 1:1 | 50 | 43.50 | 70.26 |
| C-5 | 16.67 | 11.29 | 33.33 | 35.42 | 1:2 | 50 | 46.71 | 56.50 |
| C-6 | 12.5 | 8.47 | 37.5 | 39.85 | 1:3 | 50 | 48.31 | 60.37 |
| C-7 | 10 | 6.77 | 40 | 42.50 | 1:4 | 50 | 49.28 | 60.50 |

**Table-5: Acetyl cholinesterase inhibitory activities of the compositions containing Terminalia chebula 50% aq ethanol extract (T.C-2) and Boswellia serrata oil (B.S-1).**

| Comp # | T.C-2 | | B.S-1 | | Ratio | Comp Dose µg/mL | % Inhibition of ACHE production | |
|---|---|---|---|---|---|---|---|---|
| | µg/mL | % Inhibition | µg/mL | % Inhibition | | | Additive (Calculated) | Observed |
| C-8 | 40 | 23.60 | 10 | 10.63 | 4:1 | 50 | 34.23 | 50.97 |
| C-9 | 37.5 | 22.13 | 12.5 | 13.28 | 3:1 | 50 | 35.41 | 41.18 |
| C-10 | 33.33 | 19.66 | 16.67 | 17.71 | 2:1 | 50 | 37.38 | 53.77 |
| C-11 | 25 | 14.75 | 25 | 26.57 | 1:1 | 50 | 41.32 | 79.00 |
| C-12 | 16.67 | 9.84 | 33.33 | 35.42 | 1:21 | 50 | 45.25 | 60.33 |
| C-13 | 12.5 | 7.38 | 37.5 | 39.85 | 1:3 | 50 | 47.22 | 61.00 |

**Table-6: Acetyl cholinesterase inhibitory activities of the compositions containing Terminalia chebula ethanol extract (T.C-3) and Boswellia serrata oil (B.S-1).**

| Com p # | T.C-3 | | B.S-1 | | Rati o | Com p Dose µg/m L | % Inhibition of ACHE production | |
|---|---|---|---|---|---|---|---|---|
| | µg/m L | % Inhibiti on | µg/m L | % Inhibiti on | | | Additive (Calculat ed) | Observ ed |
| C-15 | 40 | 28.94 | 10 | 10.63 | 4:1 | 50 | 39.57 | 50.56 |
| C-16 | 37.5 | 27.14 | 12.5 | 13.28 | 3:1 | 50 | 40.42 | 44.54 |
| C-17 | 33.33 | 24.12 | 16.67 | 17.71 | 2:1 | 50 | 41.83 | 54.17 |
| C-18 | 25 | 18.09 | 25 | 26.57 | 1:1 | 50 | 44.66 | 61.25 |
| C-20 | 12.5 | 9.05 | 37.5 | 39.85 | 1:3 | 50 | 48.89 | 71.00 |
| C-21 | 10 | 7.24 | 40 | 42.50 | 1:4 | 50 | 49.74 | 73.89 |

**Table-7: Acetyl cholinesterase inhibitory activities of the compositions containing Terminalia chebula 50% aq methanol extract (T.C-4) and Boswellia serrata oil (B.S-1).**

| Comp # | T.C-4 | | B.S-1 | | Ratio | Comp Dose µg/mL | % Inhibition of ACHE production | |
|---|---|---|---|---|---|---|---|---|
| | µg/mL | % Inhibition | µg/mL | % Inhibition | | | Additive (Calculated) | Observed |
| C-22 | 40 | 31.70 | 10 | 10.63 | 4:1 | 50 | 42.32 | 52.22 |
| C-23 | 37.5 | 29.72 | 12.5 | 13.28 | 3:1 | 50 | 43.00 | 49.41 |
| C-24 | 33.33 | 26.41 | 16.67 | 17.71 | 2:1 | 50 | 44.12 | 61.56 |
| C-25 | 25 | 19.81 | 25 | 26.57 | 1:1 | 50 | 46.38 | 60.58 |
| C-27 | 12.5 | 9.91 | 37.5 | 39.85 | 1:3 | 50 | 49.75 | 57.02 |
| C-28 | 10 | 7.92 | 40 | 42.50 | 1:4 | 50 | 50.43 | 61.67 |

**Table-8: Acetyl cholinesterase inhibitory activities of the compositions containing Terminalia chebula enriched extract (T.C-5) and Boswellia serrata oil (B.S-1).**

| Comp # | T.C-5 | | B.S-1 | | Ratio | Comp Dose µg/mL | % Inhibition of ACHE production | |
|---|---|---|---|---|---|---|---|---|
| | µg/mL | % Inhibition | µg/mL | % Inhibition | | | Additive (Calculated) | Observed |
| C-29 | 80 | 41.83 | 20 | 11.97 | 4:1 | 100 | 53.80 | 68.54 |
| C-30 | 75 | 39.22 | 25 | 14.97 | 3:1 | 100 | 54.18 | 73.93 |
| C-31 | 66.67 | 34.86 | 33.33 | 19.95 | 2:1 | 100 | 54.81 | 69.67 |
| C-32 | 50 | 26.15 | 50 | 29.93 | 1:1 | 100 | 56.08 | 69.00 |
| C-33 | 33.33 | 17.43 | 66.67 | 39.91 | 1:2 | 100 | 57.34 | 76.50 |
| C-34 | 25 | 13.07 | 75 | 44.90 | 1:3 | 100 | 57.97 | 77.90 |
| C-35 | 20 | 10.46 | 80 | 47.89 | 1:4 | 100 | 58.35 | 88.25 |

**Table-9: Acetyl cholinesterase inhibitory activities of the compositions containing Terminalia chebula enriched extract (T.C-1) and Boswellia serrata oil (B.S-2) & Terminalia chebula enriched extract (T.C-1) and Boswellia serrata oil (B.S-3)**

| Comp # | T.C-1 | | B.S-2 | | Ratio | Comp Dose µg/mL | % Inhibition of ACHE production | |
|---|---|---|---|---|---|---|---|---|
| | µg/mL | % Inhibition | µg/mL | % Inhibition | | | Additive (Calculated) | Observed |
| C-36 | 5.0 | 18.74 | 1.25 | 2.67 | 4:1 | 6.25 | 21.40 | 32.44 |
| C-37 | 4.69 | 17.57 | 1.56 | 3.33 | 3:1 | 6.25 | 20.90 | 42.86 |
| C-39 | 3.125 | 11.71 | 3.125 | 6.67 | 1:1 | 6.25 | 18.38 | 36.67 |
| C-41 | 1.56 | 5.85 | 4.69 | 10.00 | 1:3 | 6.25 | 15.85 | 23.56 |
| | T.C-1 | | B.S-3 | | | | | |
| C-45 | 2.08 | 11.61 | 1.04 | 2.86 | 2:1 | 3.12 | 14.46 | 21.71 |
| C-46 | 1.56 | 8.71 | 1.56 | 4.29 | 1:1 | 3.12 | 12.99 | 18.48 |
| C-47 | 1.04 | 5.80 | 2.08 | 5.71 | 1:2 | 3.12 | 11.52 | 22.58 |

### Example 20: General procedure for Inhibition of ROS production:

Anti-oxidant activity of the test sample was evaluated by measuring reactive oxygen species (ROS) scavenging activity. Reactive Oxygen Species (ROS) inhibition was estimated in HL-60 human monocytic cells. Briefly, HL-60 cells were cultured in RPMI (Sigma Cat# R8005-10X1L) media supplemented with 10% Fetal Bovine Serum (FBS; ATCC Cat# 30-2020). Cells were harvested from log phase cultures by centrifugation (270xg, 5min); they were washed (270xg, 5min) once with 1XHBSS and were collected into a 50ml falcon tube. DCF-DA (working concentration, 5µM) was added to the falcon tube containing the HL-60 cells and incubated for 15 min at 37°C in a CO₂ incubator. After the incubation, 50µl of cell suspension (125000 cells/well) was added in a 96-well black plate. Immediately, cells were either stimulated with 100nM PMA in presence or absence of different concentrations of test samples. Perkin-Elmer EnSpire plate reader was set to kinetic mode and the relative fluorescence intensity was measured at wavelengths of Ex: 460nm/Em: 550nm for every 20 min. up to 180 min. Percent of ROS inhibition was calculated by following method.

### Calculation:

To calculate the percentage of ROS inhibition, area under the curve (AUC) value has been calculated from Relative Fluorescence Intensity (RFU) vs. time plot using sigma plot software.

Normalized AUC values of each treatment were derived by deducting the AUC value of Vehicle control from treatment AUC values.

% of ROS inhibition = {(Normalized AUC value in PMA induction)-(Normalized AUC value in test sample)}/{normalized AUC value in PMA induction} x 100. The results of ROS inhibition of the individual extracts and their compositions are summarized in **Tables 10-14.**

**Table-10: ROS inhibitory activities of the compositions containing Terminalia chebula water extract (T.C-1) and Boswellia serrata oil (B.S-1).**

| Comp # | T.C-1 | | B.S-1 | | Ratio | Comp Dose µg/mL | % ROS inhibition | |
|---|---|---|---|---|---|---|---|---|
| | µg/mL | % Inhibition | µg/mL | % Inhibition | | | Additive (Calculated) | Observed |
| C-1 | 4.0 | 32.58 | 1.0 | 0.11 | 4:1 | 5 | 32.69 | 40.76 |
| C-2 | 3.75 | 30.54 | 1.25 | 0.14 | 3:1 | 5 | 30.68 | 42.18 |
| C-3 | 3.33 | 27.12 | 1.67 | 0.18 | 2:1 | 5 | 27.30 | 34.23 |
| C-4 | 2.5 | 20.36 | 2.5 | 0.28 | 1:1 | 5 | 20.64 | 42.77 |
| C-5 | 1.67 | 13.60 | 3.33 | 0.37 | 1:2 | 5 | 13.97 | 29.1 |
| C-6 | 1.25 | 10.18 | 3.75 | 0.41 | 1:3 | 5 | 10.59 | 29.93 |
| C-7 | 1.0 | 8.14 | 4.0 | 0.44 | 1:4 | 5 | 8.58 | 24.06 |

**Table-11: ROS inhibitory activities of the compositions containing Terminalia chebula 50% aq ethanol extract (T.C-2) and Boswellia serrata oil (B.S-1).**

| Comp # | T.C-2 | | B.S-1 | | Ratio | Comp Dose µg/mL | % ROS inhibition | |
|---|---|---|---|---|---|---|---|---|
| | µg/mL | % Inhibition | µg/mL | % Inhibition | | | Additive (Calculated) | Observed |
| C-8 | 4.0 | 39.39 | 1.0 | 0.11 | 4:1 | 5 | 39.50 | 50.76 |
| C-9 | 3.75 | 36.93 | 1.25 | 0.14 | 3:1 | 5 | 37.07 | 49.48 |
| C-10 | 3.33 | 32.79 | 1.67 | 0.18 | 2:1 | 5 | 32.98 | 38.43 |
| C-11 | 2.5 | 24.62 | 2.5 | 0.28 | 1:1 | 5 | 24.90 | 33.78 |
| C-12 | 1.67 | 16.45 | 3.33 | 0.37 | 1:2 | 5 | 16.81 | 39.5 |
| C-13 | 1.25 | 12.31 | 3.75 | 0.41 | 1:3 | 5 | 12.72 | 36.64 |
| C-14 | 1.0 | 9.85 | 4.0 | 0.44 | 1:4 | 5 | 10.29 | 33.44 |

**Table-12: ROS inhibitory activities of the compositions containing Terminalia chebula ethanol extract (T.C-3) and Boswellia serrata oil (B.S-1).**

| Comp # | T.C-3 | | B.S-1 | | Ratio | Comp Dose µg/mL | % ROS inhibition | |
|---|---|---|---|---|---|---|---|---|
| | µg/mL | % Inhibition | µg/mL | % Inhibition | | | Additive (Calculated) | Observed |
| C-15 | 4.0 | 37.08 | 1.0 | 0.11 | 4:1 | 5 | 37.19 | 49.02 |
| C-17 | 3.33 | 30.87 | 1.67 | 0.18 | 2:1 | 5 | 31.05 | 37.21 |
| C-18 | 2.5 | 23.18 | 2.5 | 0.28 | 1:1 | 5 | 23.45 | 39.68 |
| C-19 | 1.67 | 15.48 | 3.33 | 0.37 | 1:2 | 5 | 15.85 | 35.94 |
| C-20 | 1.25 | 11.59 | 3.75 | 0.41 | 1:3 | 5 | 12.00 | 34.41 |
| C-21 | 1.0 | 9.27 | 4.0 | 0.44 | 1:4 | 5 | 9.71 | 34.51 |

**Table-13: ROS inhibitory activities of the compositions containing Terminalia chebula 50% aq methanol extract (T.C-4) and Boswellia serrata oil (B.S-1).**

| Com p # | T.C-4 | | B.S-1 | | Rati o | Com p Dose µg/m L | % ROS inhibition | |
|---|---|---|---|---|---|---|---|---|
| | µg/m L | % Inhibiti on | µg/m L | % Inhibiti on | | | Additive (Calculat ed) | Observ ed |
| C-22 | 4.0 | 31.75 | 1.0 | 0.11 | 4:1 | 5 | 31.86 | 49.44 |
| C-23 | 3.75 | 29.77 | 1.25 | 0.14 | 3:1 | 5 | 29.91 | 39.44 |
| C-24 | 3.33 | 26.43 | 1.67 | 0.18 | 2:1 | 5 | 26.62 | 34.00 |
| C-25 | 2.5 | 19.85 | 2.5 | 0.28 | 1:1 | 5 | 20.12 | 35.18 |
| C-26 | 1.67 | 13.26 | 3.33 | 0.37 | 1:2 | 5 | 13.62 | 26.47 |
| C-27 | 1.25 | 9.92 | 3.75 | 0.41 | 1:3 | 5 | 10.34 | 23.41 |
| C-28 | 1.0 | 7.94 | 4.0 | 0.44 | 1:4 | 5 | 8.38 | 27.15 |

**Table-14: ROS inhibitory activities of the compositions containing Terminalia chebula enriched extract (T.C-5) and Boswellia serrata oil (B.S-1).**

| Comp # | T.C-5 | | B.S-1 | | Ratio | Comp Dose µg/mL | % ROS inhibition | |
|---|---|---|---|---|---|---|---|---|
| | µg/mL | % Inhibition | µg/mL | % Inhibition | | | Additive (Calculated) | Observed |
| C-29 | 4.0 | 36.31 | 1.0 | 0.11 | 4:1 | 5 | 36.42 | 46.32 |
| C-32 | 2.5 | 22.70 | 2.5 | 0.28 | 1:1 | 5 | 22.97 | 39.33 |
| C-33 | 1.67 | 15.16 | 3.33 | 0.37 | 1:2 | 5 | 15.53 | 34.4 |
| C-34 | 1.25 | 11.35 | 3.75 | 0.41 | 1:3 | 5 | 11.76 | 32.09 |
| C-35 | 1.0 | 9.08 | 4.0 | 0.44 | 1:4 | 5 | 9.52 | 29.5 |

### Example 21: General procedure for Inhibition of NADPH oxidase (NOX):

NADPH Oxidase assay was performed in differentiated HL-60 (ATCC, Manassas, VA; Cat# CCL240) human promyelocytic leukemia cells. For differentiation, HL-60 cells were collected from the T-75 culture flasks, washed and replenished with 20 mL of differentiating medium consisting of RPMI (Hi-Media Laboratories, Mumbai, India; Cat# R8005) supplemented with 10%FBS and 1.25% DMSO and incubated at 37°C in a CO₂ incubator for 7 days. Cells were harvested from the flasks and cryopreserved (5×10⁶ cells/mL) in liquid nitrogen for future use. Differentiated HL-60 cells were thawed and washed with buffer consisting of 10mL of 1XHBSS and 15mM HEPES. Cells were resuspended in 1XHBSS containing 20mM L012 at a density of 1×10⁶ cells/mL and kept aside for later use. Twenty five microliters of test samples (containing 0.2% DMSO final concentration) at required concentrations and 50µL of prepared cell suspension (50000 cells/well) were added to a white opaque 96-well plate. Immediately, luminescence was recorded at 37°C for 20 minutes with 2-minute intervals in a multi-mode reader (Spectramax 2e, San Jose, CA, USA). The plate was removed and 25µL of 400nM PMA (prepared in 1XHBSS) was added to all the wells except for control (cells only). Luminescence was recorded at 37°C for 20 minutes with 2-minute intervals. Area under curve (AUC) was calculated for each treatment to determine the percent inhibition.

### Calculation:

% NOX inhibition = {(AUC of normalized induction)-( AUC of normalized treatments)}/{AUC of normalized induction} x 100. The results of NOX inhibition of the individual extracts and their compositions are summarized in Tables 15-21.

**Table-15: NADPH (NOX) inhibitory activities of the compositions containing Terminalia chebula water extract (T.C-1) and Boswellia serrata oil (B.S-1).**

| Comp # | T.C-1 | | B.S-1 | | Ratio | Comp Dose µg/mL | % NOX inhibition | |
|---|---|---|---|---|---|---|---|---|
| | µg/mL | % Inhibition | µg/mL | % Inhibition | | | Additive (Calculated) | Observed |
| C-1 | 400 | 67.37 | 100 | 3.29 | 4:1 | 500 | 70.66 | 85.44 |
| C-2 | 375 | 63.16 | 125 | 4.12 | 3:1 | 500 | 67.28 | 85.13 |
| C-3 | 333.3 3 | 56.14 | 166.6 7 | 5.49 | 2:1 | 500 | 61.63 | 81.81 |
| C-4 | 250 | 42.11 | 250 | 8.24 | 1:1 | 500 | 50.34 | 80.19 |
| C-5 | 166.6 7 | 28.07 | 333.3 3 | 10.98 | 1:2 | 500 | 39.05 | 66.72 |
| C-6 | 125 | 21.05 | 375 | 12.35 | 1:3 | 500 | 33.41 | 61.2 |
| C-7 | 100 | 16.84 | 400 | 13.18 | 1:4 | 500 | 30.02 | 56.73 |

**Table-16: NADPH (NOX) inhibitory activities of the compositions containing Terminalia chebula 50% aq ethanol extract (T.C-2) and Boswellia serrata oil (B.S-1).**

| Comp # | T.C-2 | | B.S-1 | | Ratio | Comp Dose µg/mL | % NOX inhibition | |
|---|---|---|---|---|---|---|---|---|
| | µg/mL | % Inhibition | µg/mL | % Inhibition | | | Additive (Calculated) | Observed |
| C-8 | 400 | 69.88 | 100 | 3.29 | 4:1 | 500 | 73.17 | 84.79 |
| C-9 | 375 | 65.51 | 125 | 4.12 | 3:1 | 500 | 69.63 | 82.02 |
| C-10 | 333.33 | 58.23 | 166.67 | 5.49 | 2:1 | 500 | 63.72 | 80.47 |
| C-11 | 250 | 43.68 | 250 | 8.24 | 1:1 | 500 | 51.92 | 77.1 |
| C-12 | 166.67 | 29.12 | 333.33 | 10.98 | 1:2 | 500 | 40.10 | 72.83 |
| C-13 | 125 | 21.84 | 375 | 12.35 | 1:3 | 500 | 34.19 | 65.28 |
| C-14 | 100 | 17.47 | 400 | 13.18 | 1:4 | 500 | 30.65 | 57.95 |

**Table-17: NADPH (NOX) inhibitory activities of the compositions containing Terminalia chebula ethanol extract (T.C-3) and Boswellia serrata oil (B.S-1).**

| | T.C-3 | | B.S-1 | | | | % NOX inhibition | |
|---|---|---|---|---|---|---|---|---|
| Comp # | µg/mL | % Inhibition | µg/mL | % Inhibition | Ratio | Comp Dose µg/mL | Additive (Calculated) | Observed |
| C-15 | 400 | 68.44 | 100 | 3.29 | 4:1 | 500 | 71.73 | 84.3 |
| C-16 | 375 | 64.16 | 125 | 4.12 | 3:1 | 500 | 68.28 | 79.65 |
| C-17 | 333.33 | 57.03 | 166.67 | 5.49 | 2:1 | 500 | 62.52 | 77.2 |
| C-18 | 250 | 42.78 | 250 | 8.24 | 1:1 | 500 | 51.01 | 79.01 |
| C-19 | 166.67 | 28.52 | 333.33 | 10.98 | 1:2 | 500 | 39.50 | 61.06 |
| C-20 | 125 | 21.39 | 375 | 12.35 | 1:3 | 500 | 33.74 | 59.91 |
| C-21 | 100 | 17.11 | 400 | 13.18 | 1:4 | 500 | 30.29 | 57.76 |

**Table-18: NADPH (NOX) inhibitory activities of the compositions containing Terminalia chebula 50% aq methanol extract (T.C-4) and Boswellia serrata oil (B.S-1).**

| Comp # | T.C-4 | | B.S-1 | | Ratio | Comp Dose µg/mL | % NOX inhibition | |
|---|---|---|---|---|---|---|---|---|
| | µg/mL | % Inhibition | µg/mL | % Inhibition | | | Additive (Calculated) | Observed |
| C-22 | 400 | 68.21 | 100 | 3.29 | 4:1 | 500 | 71.50 | 87.96 |
| C-23 | 375 | 63.95 | 125 | 4.12 | 3:1 | 500 | 68.06 | 85.77 |
| C-24 | 333.33 | 56.84 | 166.67 | 5.49 | 2:1 | 500 | 62.33 | 86.69 |
| C-25 | 250 | 42.63 | 250 | 8.24 | 1:1 | 500 | 50.87 | 78.86 |
| C-26 | 166.67 | 28.42 | 333.33 | 10.98 | 1:2 | 500 | 39.40 | 74.58 |
| C-27 | 125 | 21.32 | 375 | 12.35 | 1:3 | 500 | 33.67 | 69.39 |
| C-28 | 100 | 17.05 | 400 | 13.18 | 1:4 | 500 | 30.23 | 64.16 |

**Table-19: NADPH (NOX) inhibitory activities of the compositions containing Terminalia chebula enriched extract (T.C-5) and Boswellia serrata oil (B.S-1).**

| Comp # | T.C-5 | | B.S-1 | | Ratio | Comp Dose µg/mL | % NOX inhibition | |
|---|---|---|---|---|---|---|---|---|
| | µg/mL | % Inhibition | µg/mL | % Inhibition | | | Additive (Calculated) | Observed |
| C-29 | 400 | 70.54 | 100 | 3.29 | 4:1 | 500 | 73.84 | 85.21 |
| C-30 | 375 | 66.14 | 125 | 4.12 | 3:1 | 500 | 70.25 | 84.03 |
| C-31 | 333.33 | 58.79 | 166.67 | 5.49 | 2:1 | 500 | 64.28 | 84.87 |
| C-32 | 250 | 44.09 | 250 | 8.24 | 1:1 | 500 | 52.33 | 81.62 |
| C-33 | 166.67 | 29.39 | 333.33 | 10.98 | 1:2 | 500 | 40.37 | 68.5 |
| C-34 | 125 | 22.05 | 375 | 12.35 | 1:3 | 500 | 34.40 | 64.2 |
| C-35 | 100 | 17.64 | 400 | 13.18 | 1:4 | 500 | 30.81 | 63.14 |

**Table-20: NADPH (NOX) activities of the compositions containing Terminalia chebula enriched extract (T.C-1) and Boswellia serrata oil (B.S-2)**

| Comp # | T.C-1 | | B.S-2 | | Ratio | Comp Dose µg/mL | % NOX inhibition | |
|---|---|---|---|---|---|---|---|---|
| | µg/mL | % Inhibition | µg/mL | % Inhibition | | | Additive (Calculated) | Observed |
| C-36 | 400 | 67.37 | 100 | 0.57 | 4:1 | 500 | 67.94 | 83.4 |
| C-37 | 375 | 63.16 | 125 | 0.72 | 3:1 | 500 | 63.88 | 82.95 |
| C-38 | 333.3 3 | 56.14 | 166.6 7 | 0.96 | 2:1 | 500 | 57.10 | 68.26 |
| C-39 | 250 | 42.11 | 250 | 1.44 | 1:1 | 500 | 43.54 | 78.27 |
| C-40 | 166.67 | 28.07 | 333.33 | 1.91 | 1:2 | 500 | 29.98 | 55.07 |
| C-41 | 125 | 21.05 | 375 | 2.15 | 1:3 | 500 | 23.21 | 62.92 |
| C-42 | 100 | 16.84 | 400 | 2.30 | 1:4 | 500 | 19.14 | 43.51 |

**Table-21: NADPH (NOX) activities of the compositions containing Terminalia chebula enriched extract (T.C-1) and Boswellia serrata oil (B.S-3)**

| Comp # | T.C-1 | | B.S-3 | | Ratio | Comp Dose µg/mL | % NOX inhibition | |
|---|---|---|---|---|---|---|---|---|
| | µg/mL | % Inhibition | µg/mL | % Inhibition | | | Additive (Calculated) | Observed |
| C-43 | 400 | 67.37 | 100 | 1.16 | 4:1 | 500 | 68.52 | 89.04 |
| C-44 | 375 | 63.16 | 125 | 1.45 | 3:1 | 500 | 64.60 | 86.86 |
| C-45 | 333.33 | 56.14 | 166.67 | 1.93 | 2:1 | 500 | 58.07 | 86.03 |
| C-46 | 250 | 42.11 | 250 | 2.89 | 1:1 | 500 | 45.00 | 76.99 |
| C-47 | 166.67 | 28.07 | 333.33 | 3.85 | 1:2 | 500 | 31.92 | 72.3 |
| C-48 | 125 | 21.05 | 375 | 4.34 | 1:3 | 500 | 25.39 | 70.03 |
| C-49 | 100 | 16.84 | 400 | 4.62 | 1:4 | 500 | 21.47 | 77.68 |

### Example 22: Evaluation of the composition for Nootropic activity (Learning and Memory enhancement and stress) in Morris Water Maze and T-maze

**Treatments:** Twenty four healthy male Sprague-Dawley rats were selected and randomly assigned to four groups (n=6). The animals were supplemented (using oral gavage) with either 150 mg/kg of B.S-1 (G2) or T.C-1 (G3) or comp-50 (G4) in 10 mL of 0.5% CMC in water, for 32 days. The rats in the control (G1) group received only the vehicle (10 mL of 0.5% CMC in water) during the study. Morris water maze test was conducted on all animals between the day 22 and day 26; the T-maze test was performed on between day 29 and day 31 of the study. On day 32, prior to blood collection animals were fasted for 4 hours; after blood collection the rats were euthanized using excess CO2. Brain tissues were collected and preserved for estimation of total choline levels.

**Test parameters:** Target latency, distanced travelled, number of target crossings; working memory index were evaluated as per the procedures disclosed in the literature; serum corticosterone and total choline in brain homogenate using the test kits as per the procedures suggested by the kit manufacturers.

The target latency and the travelled distance were significantly improved in the rats supplemented with the test substances, when compared to the control rats. In addition, these groups supplemented with the test substances also showed increased number of target crossings in comparison with the control. Further, the rats supplemented with composition-50 shows sunbstantially increased number of target crossings in comparison with the rats supplemented with Boswellia serrata oil (B.S-1), Terminalia chebula water extract (T.C-1) Together, these observations indicate that the supplementation with composition-50 synergistically improves spatial learning in the rats(figure II).

## Claims

1. Non-medical use of a synergistic herbal composition for improving at least one cognitive function selected from memory, intelligence, learning, retention, recall information/communication, focus, concentration, attention, perception, reasoning, problem solving, decision making and mental fatigue, the composition comprising a first ingredient selected from the extract(s), fraction(s), phytochemical(s) and mixtures thereof derived from Terminalia chebula in combination with a second ingredient selected from the oil extract(s)/fraction(s) derived from Boswellia serrata gum resin, after removing boswellic acids,
wherein the first ingredient is a Terminalia chebula fruit extract produced using at least one solvent selected from ethanol, methanol, water and mixtures thereof, wherein the second ingredient is produced by extracting Boswellia serrata gum resin with at least one solvent selected from ethanol, ethyl acetate, methyl isobutyl ketone and mixtures thereof, and washing with an inorganic base.

2. The non-medical use as claimed in claim 1, wherein the composition contains at least one additional component selected from the group consisting of Nootropic agent(s); pharmaceutically acceptable active ingredients, vitamins, minerals; pharmaceutically or nutraceutically or dietically acceptable, excipients, carriers or diluents.

3. The non-medical use as claimed in claim 2, wherein the pharmaceutically or nutraceutically or dietically acceptable excipients, carriers and diluents are selected from glucose, dextrose, fructose, galactose, sucrose, maltose, lactose, lactulose, trehalose cellobiose, chitobiose, starch, sodium starch glycolate, pre gelatinized starch, soluble starch, Ultrasperse A & Ultra-tex 4, yellow dextrin, white dextrin, maltodextrin, sorbitol, mannitol, inositol, xylitol, isomalt, microcrystalline cellulose, hydroxy propyl methyl cellulose, hydroxy ethyl cellulose, sodium carboxy methyl cellulose (sodium CMC), neusilin, veegum, talc, colloidal silicon dioxide, calcium stearate, magnesium stearate, zinc stearate, citric acid, tartaric acid, malic acid, succinic acid, lactic acid, L-ascorbic acid, fatty acid esters and esters of poly sorbate, acacia, carrageenan, guar gum, xanthan gum, vitamin B group, nicotinamide, calcium pantothenate, amino acids, casein, gelatin, pectin, agar, sodium chloride, calcium chloride, dicalcium phosphate, magnesium hydroxide, zinc sulphate, zinc chloride, natural pigments, flavors, Class I & Class Il preservatives and aqueous, alcoholic, hydro-alcoholic, organic solutions of above listed ingredients alone or in combination.

4. The non-medical use as claimed in claims 1 or 2, wherein the composition is formulated into a dosage form selected from dry powder form, liquid form, beverage, food product, dietary supplement or any suitable form such as tablet, a capsule, a soft chewable tablet or gummy bear.

5. The non-medical use as claimed in claims 1 to 3, wherein the composition is formulated into nutritional/dietary supplements that can be contemplated/made into the dosage form of healthy foods, or food for specified health uses such as solid food like chocolate or nutritional bars, semisolid food like cream, jam, or gel or beverage such as refreshing beverage, lactic acid bacteria beverage, drop, candy, chewing gum, gummy candy, yoghurt, ice cream, pudding, soft adzuki bean jelly, jelly, cookie, tea, soft drink, juice, milk, coffee, cereal, snack bar.

## Patentansprüche

1. Nichtmedizinische Verwendung einer synergistischen pflanzlichen Zusammensetzung zur Verbesserung von mindestens einer kognitiven Funktion, ausgewählt aus Erinnerungsvermögen, Intelligenz, Lernen, Gedächtnis, Abruf von Informationen / Kommunikation, Orientierung, Konzentration, Aufmerksamkeit, Wahrnehmung, logischem Denken, Problemlösung, Entscheidungsfindung und mentaler Erschöpfung, wobei die Zusammensetzung einen ersten Bestandteil, ausgewählt aus dem/den Extrakt(en), der/den Fraktion(en), der/den Phytochemikalie(n) und den Gemischen davon, abgeleitet von Terminalia chebula, in Kombination mit einem zweiten Bestandteil, ausgewählt aus dem/den Ölextrakt(en) / der/den Ölfraktion(en), abgeleitet von Gummiharz von Boswellia serrata, nach dem Entfernen von Boswelliasäuren, umfasst,
wobei der erste Bestandteil ein Extrakt von Früchten von Terminalia chebula ist, hergestellt unter Verwendung von mindestens einem Lösungsmittel, ausgewählt aus Ethanol, Methanol, Wasser und Gemischen davon,
wobei der zweite Bestandteil durch Extrahieren von Gummiharz von Boswellia serrata mit mindestens einem Lösungsmittel, ausgewählt aus Ethanol, Ethylacetat, Methylisobutylketon und Gemischen davon, und Waschen mit einer anorganischen Base hergestellt wird.

2. Nichtmedizinische Verwendung nach Anspruch 1, wobei die Zusammensetzung mindestens eine weitere Komponente, ausgewählt aus der Gruppe bestehend aus einem oder mehreren nootropischen Wirkstoffen; pharmazeutisch akzeptablen Wirkstoffen, Vitaminen, Mineralien; pharmazeutisch oder nutrazeutisch oder diätetisch akzeptablen Exzipienten, Trägern oder Verdünnungsmitteln, enthält.

3. Nichtmedizinische Verwendung nach Anspruch 2, wobei die pharmazeutisch oder nutrazeutisch oder diätetisch akzeptablen Exzipienten, Träger und Verdünnungsmittel ausgewählt sind aus Glucose, Dextrose, Fructose, Galactose, Saccharose, Maltose, Lactose, Lactulose, Trehalose, Cellobiose, Chitobiose, Stärke, Natriumstärkeglykolat, vorgelatinierter Stärke, löslicher Stärke, Ultra-Sperse A & Ultra-Tex 4, Gelbdextrin, Weißdextrin, Maltodextrin, Sorbitol, Mannitol, Inositol, Xylitol, Isomalt, mikrokristalliner Cellulose, Hydroxypropylmethylcellulose, Hydroxyethylcellulose, Natriumcarboxymethylcellulose (Natrium-CMC), Neusilin, Veegum, Talkum, kolloidalem Siliciumdioxid, Calciumstearat, Magnesiumstearat, Zinkstearat, Citronensäure, Weinsäure, Äpfelsäure, Bernsteinsäure, Milchsäure, L-Ascorbinsäure, Fettsäureestern und Estern von Polysorbat, Acacia, Carrageenan, Guaran, Xanthan, Vitamin-B-Gruppe, Nicotinamid, Calciumpantothenat, Aminosäuren, Casein, Gelatine, Pektin, Agar, Natriumchlorid, Calciumchlorid, Calciumhydrogenphosphat, Magnesiumhydroxid, Zinksulfat, Zinkchlorid, natürlichen Pigmenten, Aromen, Konservierungsstoffen der Klasse I und Klasse II und wässrigen, alkoholischen, wässrig-alkoholischen, organischen Lösungen der vorstehend aufgeführten Inhaltsstoffe allein oder in Kombination.

4. Nichtmedizinische Verwendung nach den Ansprüchen 1 oder 2, wobei die Zusammensetzung in eine Dosierungsform, ausgewählt aus Trockenpulverform, flüssiger Form, Getränk, Lebensmittelprodukt, Nahrungsergänzungsmittel oder einer geeigneten Form wie Tablette, einer Kapsel, einer weichen Kautablette oder einem weichen Gummibärchen, formuliert wird.

5. Nichtmedizinische Verwendung nach den Ansprüchen 1 bis 3, wobei die Zusammensetzung in Nahrungsergänzungsmittel formuliert wird, die in die Dosierungsform von gesunden Lebensmitteln oder Lebensmitteln für spezifizierte gesundheitliche Verwendungen erwogen/hergestellt werden können, wie z. B. feste Lebensmittel wie Schokoladen- oder Ernährungsriegel, halbfeste Lebensmittel wie Creme, Marmelade oder Gel, oder Getränke wie Erfrischungsgetränk, Milchsäurebakterien-Getränk, Drops, Bonbons, Kaugummi, Gummisüßigkeit, Joghurt, Eiscreme, Pudding, weiches Adzukibohnengelee, Gelee, Plätzchen, Tee, Softdrink, Saft, Milch, Kaffee, Cerealie, Snackriegel.

## Revendications

1. Utilisation non médicale d'une composition synergique à base de plantes pour améliorer au moins une fonction cognitive choisie parmi la mémoire, l'intelligence, l'apprentissage, la rétention, le rappel d'informations/la communication, la focalisation, la concentration, l'attention, la perception, le raisonnement, la résolution de problèmes, la prise de décision et la fatigue mentale, la composition comprenant un premier ingrédient choisi parmi le(s) extrait(s), la/les fraction(s), le(s) composé(s) phytochimique(s) et leurs mélanges dérivés de *Terminalia chebula,* en combinaison avec un deuxième ingrédient choisi parmi le(s) extrait(s) huileux/la/les fraction(s) huileuse(s) dérivés de résine de gomme de *Boswellia serrata,* après élimination des acides boswelliques,
dans laquelle le premier ingrédient est un extrait de fruit de *Terminalia chebula* produit à l'aide d'au moins un solvant choisi parmi l'éthanol, le méthanol, l'eau et leurs mélanges,
dans laquelle le deuxième ingrédient est produit par extraction de résine de gomme de Boswellia serrata avec au moins un solvant choisi parmi l'éthanol, l'acétate d'éthyle, la méthylisobutylcétone et leurs mélanges, et lavage avec une base inorganique.

2. Utilisation non médicale selon la revendication 1, dans laquelle la composition contient au moins un composant supplémentaire choisi dans le groupe constitué par un ou plusieurs agents nootropes , des principes actifs pharmaceutiquement acceptables, des vitamines, des minéraux ; des excipients, véhicules ou diluants pharmaceutiquement, nutraceutiquement ou diététiquement acceptables.

3. Utilisation non médicale selon la revendication 2, dans laquelle les excipients, véhicules et diluants pharmaceutiquement, nutraceutiquement ou diététiquement acceptables sont choisis parmi le glucose, le dextrose, le fructose, le galactose, le saccharose, le maltose, le lactose, le lactulose, le tréhalose, la cellobiose, le chitobiose, l'amidon, le glycolate d'amidon sodique, l'amidon prégélatinisé, l'amidon soluble, l'Ultrasperse A et l'Ultra-tex 4, la dextrine jaune, la dextrine blanche, la maltodextrine, le sorbitol, le mannitol, l'inositol, le xylitol, l'isomalt, la cellulose microcristalline, l'hydroxypropylméthylcellulose, l'hydroxyéthylcellulose, la carboxyméthylcellulose sodique (CMC sodique), le Neusilin, le Veegum, le talc, le dioxyde de silicium colloïdal, le stéarate de calcium, le stéarate de magnésium, le stéarate de zinc, l'acide citrique, l'acide tartrique, l'acide malique, l'acide succinique, l'acide lactique, l'acide L-ascorbique, les esters d'acides gras et les esters de polysorbate, l'acacia, le carraghénane, la gomme de guar, la gomme xanthane, les vitamines du groupe B, le nicotinamide, le pantothénate de calcium, les acides aminés, la caséine, la gélatine, la pectine, l'agar, le chlorure de sodium, le chlorure de calcium, le phosphate dicalcique, l'hydroxyde de magnésium, le sulfate de zinc, le chlorure de zinc, des pigments naturels, des arômes, des conservateurs de classe I et de classe II, ainsi que des solutions aqueuses, alcooliques, hydroalcooliques, organiques des ingrédients énumérés ci-dessus, seuls ou en combinaison.

4. Utilisation non médicale selon les revendications 1 ou 2, dans laquelle la composition est formulée sous une forme posologique choisie parmi une forme de poudre sèche, une forme liquide, une boisson, un produit alimentaire, un complément alimentaire ou toute forme appropriée telle que comprimé, une gélule, un comprimé à mâcher tendre ou un ourson gélifié.

5. Utilisation non médicale selon les revendications 1 à 3, dans laquelle la composition est formulée sous la forme de compléments nutritionnels/diététiques pouvant être envisagés/réalisés sous la forme posologique d'aliments sains ou d'aliments destinés à des usages de santé spécifiques, tels que des aliments solides tels que du chocolat ou des barres nutritionnelles, des aliments semi-solides tels que de la crème, de la confiture ou un gel, ou des boissons telles qu'une boisson rafraîchissante, une boisson à base de bactéries lactiques, des gouttes, des bonbons, du chewing-gum, des bonbons gélifiés, du yaourt, de la crème glacée, du pudding, de la gelée de haricots adzuki tendre, de la gelée, des biscuits, du thé, une boisson gazeuse, du jus, du lait, du café, des céréales ou une barre de collation.
